(19) Europäisches Patentamt
European Patent Office
Office européen des brevets

(11) EP 1 871 333 B1

(12) EUROPÄISCHE PATENTSCHRIFT

(45) Veröffentlichungstag und Bekanntmachung des Hinweises auf die Patenterteilung:
**05.10.2011 Patentblatt 2011/40**

(21) Anmeldenummer: **06724430.1**

(22) Anmeldetag: **19.04.2006**

(51) Int Cl.:
***A61K 6/093*** (2006.01) ***C07F 7/18*** (2006.01)
***C08G 77/20*** (2006.01) ***C08G 77/26*** (2006.01)

(86) Internationale Anmeldenummer:
**PCT/EP2006/003585**

(87) Internationale Veröffentlichungsnummer:
**WO 2006/111373 (26.10.2006 Gazette 2006/43)**

(54) **WIDERSTANDSFÄHIGE, LANGLEBIGE, DENTALE KOMPOSITE**

TOUGH, LONG-LASTING DENTAL COMPOSITES

COMPOSITE DENTAIRE DURABLE RESISTANT

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **20.04.2005 DE 102005018351**

(43) Veröffentlichungstag der Anmeldung:
**02.01.2008 Patentblatt 2008/01**

(73) Patentinhaber: **Fraunhofer-Gesellschaft zur Förderung der angewandten Forschung e.V.
80686 München (DE)**

(72) Erfinder:
- **WOLTER, Herbert
97941 Tauberbischofsheim (DE)**
- **GELLERMANN, Carsten
97218 Gerbrunn (DE)**
- **STORCH, Werner
97206 Hoechberg (DE)**

(74) Vertreter: **Olgemöller, Luitgard Maria et al
Patentanwältin
Lindenstraße 12a
81545 München (DE)**

(56) Entgegenhaltungen:
**EP-A- 0 451 709**
**EP-A- 0 804 919**
**EP-A- 1 022 012**
**WO-A-01/08639**
**WO-A-2005/040249**
**DE-C1- 4 416 857**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 871 333 B1

**Beschreibung**

Die vorliegende Erfindung betrifft dentale Komposite mit verbesserten Eigenschaften. Die Komposite werden unter Verwendung von Füllstoffen und Kieselsäureheteropolykondensaten hergestellt, die ihrerseits zumindest teilweise aus einem Grundgerüst bestehen, das mindestens zwei (Meth-)Acrylatreste pro Silyleinheit aufweist, wobei mindestens einer dieser (Meth-)Acrylatreste über eine Urethangruppe gebunden vorliegt. Für die vorliegende Erfindung geeignete Ausgangsmaterialien (Silanverbindungen) sind unter anderen Verbindungen in der DE 103 49 766.8 beschrieben, die zum Zeitpunkt der Einreichung der vorliegenden Anmeldung der Öffentlichkeit noch nicht zugänglich gemacht wurde.

In der Vergangenheit wurde eine Vielzahl von plastisch verarbeitbaren Kompositen auf Basis von organischen Monomeren (z.B. Mono-, Di-, Tri- oder noch höheren Methacrylaten) in Verbindung mit kommerziell erhältlichen Füllstoffen (z.B. röntgenopaken Dentalgläsern, hochdispersen Kieselsäuren oder dergleichen) für den Einsatz als dentale Restaurationsmaterialien entwickelt, welche sich erfolgreich am Markt behauptet haben. Zu den teilweise aber dennoch nicht befriedigenden physikalischen Eigenschaften zählen z.B. eine zu hohe Abrasion, eine zu hohe Härtungsschrumpfung, die zu einer Randspaltbildung insbesondere unter Kaubelastung führt, eine zu geringe Röntgenabsoption, ästhetische Mängel sowie ein allergenes Potential (bedingt durch Restmonomere). Erste wesentliche Fortschritte in dieser Hinsicht wurden mit dem Einsatz von anorganisch-organischen Hybridpolymeren (ORMOCER®en) erzielt, bedingt durch deren dualen Charakter als Matrixsysteme. Entsprechende Komposite für den dentalen Einsatz werden z.B. in der Patentanmeldung DE 41 33 494 beschrieben. Somit ist man in der Lage, viele wichtige Einzeleigenschaften/Anforderungen zu realisieren.

WO 01/08639 A1 beschäftigt sich mit Makromonomeren, die durch Hydrolyse und Kondensation der darin enthaltenen Silylgruppen zu Zusammensetzungen verarbeitet werden können, die sich für den Dentalbereich eignen. Ein Formelschema in dieser Druckschrift zeigt Verbindungen, in denen Silylgruppen über eine Urethangruppe am Rückgrat des (linearen) Makromonomeren gebunden sind, ohne dass angegeben wäre, in welche Richtung die Urethangruppen angeordnet sein sollen. In dem einzigen Beispiel, das ein konkretes, Urethangruppen enthaltendes Makromonomer zeigt, sind diese Urethangruppen allerdings nicht zwischen dem organischen Molekül-Rückgrat und den Silylgruppen angeordnet, sondern sie alternieren mit Silylalkylenresten als Substituenten am linearen Rückgrat des Makromonomeren. Aus Anspruch 8 geht hervor, dass die Autoren dieser Druckschrift das konkret angegebene Makromonomer unter das erwähnte Formelschema mit Urethangruppen als Spacer zwischen der Silylgruppe und dem Molekül-Rückgrat subsumiert wissen wollen.

Die Realisierung aller wesentlichen Einzelanforderungen (die Schrumpfung, die Ästhetik, die Randdichtigkeit, die Abrasionsfestigkeit, die Biokompatibilität, die Röntgenopazität und ggf. weitere betreffend) in Kombination, d.h. in einem Materialtyp und auf deutlich erhöhtem Gesamteigenschaftsniveau als Voraussetzung für eine signifikant gesteigerte "Lebensdauer" von Restaurations- und Prophylaxemaßnahmen ist mit den bisherigen Materialien jedoch noch nicht gelungen. Außerdem gewinnt ein weiterer Aspekt zunehmend an Bedeutung, nämlich die Biokompatibilität der Dentalmaterialien. So können Restmonomere, die durch nicht vollständige Aushärtung der Komposite verbleiben und nachfolgend langsam aus dem Material austreten, beim Patienten zu Allergien führen. Darüber hinaus wäre es häufig wünschenswert, dass die Materialien bereits in dem Zustand, in dem sie in die Zahnarztpraxis gelangen, monomerfrei sind, um z.B. möglichen Allergien der Zahnarzthelferinnen oder des Zahnarztes vorzubeugen.

Aufgabe der vorliegenden Erfindung ist es, hier Abhilfe zu schaffen und neue Komposite sowie deren Verwendung im Dentalbereich z.B. für Restaurationen, Prophylaxemaßnahmen, Prothetik, Zahnregulierung bereitzustellen.

Gelöst wird diese Aufgabe durch die Bereitstellung von dentalen Kompositen gemäß Anspruch 1 sowie Verwendungen gemäß Anspruch 15. Es sind damit gute Voraussetzungen gegeben, dem heutigen Wunsch nach Restaurations- und Prophylaxemaßnahmen mit einer signifikant gesteigerten "Lebensdauer" bei optimaler Ästhetik und Biokompatibilität umzusetzen. Die den Kompositen zugrunde liegende hybride Matrix besteht aus (Meth)Acrylatgruppen enthaltenden Harzsystemen, die z.B. in der bereits oben erwähnten DE 103 49 766.8 beschrieben sind. Sie können entweder durch hydrolytische Kondensation von Silanverbindungen erhalten werden, die im Rahmen einer Isocyanataddition an eine OH-gruppenhaltige Verbindung hergestellt werden können. Alternativ können sie durch hydrolytische Kondensation von hydroxylgruppenhaltigen Silanverbindungen und nachträgliche Umsetzung des so erhaltenen Harzes mit einer entsprechenden, mindestens eine (Meth-)Acrylatgruppe enthaltenden Isocyanat-Verbindung gewonnen werden. Ein weiterer wesentlicher Bestandteil der Komposite neben dem Harzsystem ist ein nanopartikulärer, ggf. funktionalisierter Füllstoff, der weiter unten näher beschrieben wird.

Basis für die Harzsysteme, aus denen sich die erfindungsgemäßen Komposite erzeugen lassen, sind Strukturelemente der Formel (Ib)

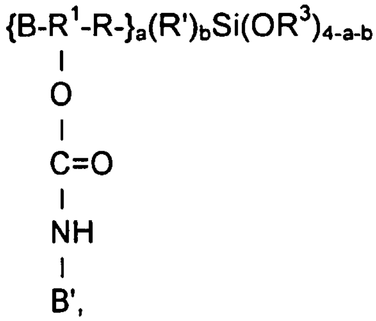

(Ib)

worin die Reste und Indices die folgende Bedeutung haben:

R ist eine offenkettige und/oder cyclische Alkylen-, Arylen- oder Alkylenarylengruppe mit jeweils 1 bis 10 Kohlenstoffatomen, die in manchen Fällen durch eine oder mehrere Sauerstoff- oder Schwefelatome oder Carboxyl- oder Aminogruppen unterbrochen sein oder solche Atome/Gruppen an ihrem dem Siliciumatom abgewandten Ende tragen kann.

$R^1$ ist eine substituierte, offenkettige und/oder cyclische Alkylen-, Arylen- oder Alkylenarylengruppe mit jeweils 1 bis 10 Kohlenstoffatomen, die in manchen Fällen durch eine oder mehrere Sauerstoff- oder Schwefelatome oder Carboxyl- oder Aminogruppen unterbrochen sein oder solche Atome/Gruppen an einem ihrer Enden tragen kann und die, wie aus der Formel ersichtlich, mit einer B' tragenden Urethangruppe substituiert ist.

R' ist eine offenkettige und/oder cyclische Alkyl-, Alkenyl-, Aryl-, Alkylaryl- oder Arylalkylgruppe mit vorzugsweise 1 bis 20 Kohlenstoffatomen; diesbezüglich sei auch auf die weiteren Erläuterungen zur Funktion dieser Gruppe verwiesen, die sich bei der Definition der unten beschriebenen Formel (II) finden.

B und B' können gleich oder verschieden sein; beide Reste haben die Bedeutung einer geradkettigen, verzweigten oder cyclischen organischen Gruppe, die mindestens einen (Meth)Acrylatrest aufweist und demzufolge mindestens 3 und vorzugsweise bis zu 50 Kohlenstoffatomen besitzt. Mindestens einer der Reste B und B' kann in einer bevorzugten Ausgestaltung neben der erwähnten ersten Acrylat- oder Methacrylatgruppe ein zweites oder sogar ein zweites und ein drittes Michaelsystem umfassen, bei dem es sich wiederum um eine Acrylat- oder Methacrylatgruppe handeln kann, aber nicht muss. Besonders zu nennen sind Reste B und B', die als Strukturelemente $C_2$-$C_4$-Alkandiole, die Trimethylolpropangruppe, die Pentaerythritgruppe oder die Glycerolstruktur enthalten. B und B' können Acrylsäureestergruppen und oder Methacrylsäureestergruppen des Trimethylolpropan, der Glycerins, des Pentaerythrits, der $C_2$-$C_4$-Alkandiole, der Polyethylenglycole, der Polypropylenglycole oder des gegebenenfalls substituierten und/oder alkoxylierten Bisphenol A sein oder diese Ester umfassen. Ebenfalls bevorzugt ist aber auch die Ausgestaltung, in der B und B' nur eine (Meth)acrylatgruppe darstellen, die über eine Esterbindung des Carboxylrestes an das übrige Molekül gebunden ist. B und B' können ein durchgehendes Kohlenstoffskelett aufweisen, die Kohlenstoffkette(n) (Haupt- und/oder Seitenkette(n) können aber auch durch Heteroatome bzw. Gruppen wie O, S, SO, NH, NHCO, PR, POR, CONHCO, COO, NHCOO oder dergleichen unterbrochen sein. Das Kohlenstoffskelett von B oder B' kann ausschließlich aliphatisch sein, und zwar mit offenen und/oder geschlossenen Strukturen, B und B' können aber auch einen oder mehrere aromatische(n) Kern(e) oder kondensierte Systeme oder Triazingruppen oder dgl. aufweisen, z.B. Bisphenol-A-Strukturen oder dergleichen. Ferner können die Gruppen oder Strukturen beliebig substituiert sein, z.B. mit Säure-, Säureamid-, Ester- oder Aminogruppen.

Die an das Siliciumatom gebundenen Reste $R^3$ können gleich oder verschieden sein. Zumindest ein Teil davon muss die Bedeutung einer Bindung zu einem anderen Siliciumatom, ggf. stattdessen teilweise auch zu einem anderen Metallatom, das sich in Kieselsäureheteropolykondensate einbauen lässt, besitzen. In vielen Fällen werden nicht alle Reste $R^3$ diese Bedeutung haben, dann wird ein Teil davon stattdessen ein Wasserstoffatom sein, das Siliciumatom also eine oder mehrere Hydroxygruppen tragen. Für den Fall, dass 4-a-b 3 ist, können durchschnittlich in etwa 30 bis 70 %, also bis zu etwa zwei der drei Gruppen $OR^3$ Hydroxy sein. Wenn 4-a-b 2 ist, kann die Anzahl der nicht vernetzten Gruppen $OR^3$ durchschnittlich bis zu etwa 50% betragen. Bei 50% ist durchschnittlich eine der beiden Gruppen mit einem weiteren Silicium- oder Metallatom vernetzt. In manchen der vorgenannten Fälle kann ein Teil der Gruppen $R^3$ statt Wasserstoff auch eine Alkylgruppe mit 1 bis 10, vorzugsweise 1 bis 4, Kohlenstoffatomen darstellen. Über den Anteil der Reste, die Bindungen zu weiteren Si-Atomen oder anderen Metallatomen darstellen, definiert sich der Kondensationsgrad des (Teil-) Kondensates zumindest soweit, wie es sich über die obige Formel (Ib) darstellen lässt.
a bedeutet 1 oder 2, vorzugsweise 1, und b kann 0 oder 1 sein.

$R^3$ ist vorzugsweise ein Rest mit 1 bis 4 Kohlenstoffatomen und besonders bevorzugt Methyl oder Ethyl, oder eine Bindung zu einem weiteren Si-Atom.

Diese Kieselsäurepolykondensate oder -teilkondensate lassen sich in einem ersten, für die vorliegende Erfindung nutzbaren Syntheseweg durch hydrolytische Kondensation aus Silanen der Formel (Ia) herstellen:

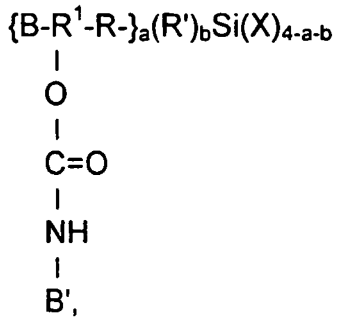

(Ia)

worin die Reste B, B', $R^1$ und R' die oben für Formel (Ib) angegebene Bedeutung besitzen und X eine Gruppe ist, die unter Ausbildung von Si-O-Si-Brücken eine hydrolytische Kondensationsreaktion eingehen kann. Gruppen X werden als anorganische Netzwerkbildner bezeichnet, da sich bei der hydrolytischen Kondensationsreaktion ein Kieselsäurepolykondensat-Netzwerk ausbilden kann. Dem Fachmann ist dementsprechend bekannt, welche Bedeutung X annehmen kann. Vorzugsweise ist X eine $C_1$-$C_{10}$-Alkoxygruppe, stärker bevorzugt eine $C_1$-$C_4$-Alkoxygruppe und ganz besonders bevorzugt Methoxy oder Ethoxy. X kann aber auch je nach Bedarf ein Halogenid wie Cl, Wasserstoff, Hydroxy, Acyloxy mit vorzugsweise 2 bis 5 Kohlenstoffatomen, Alkylcarbonyl mit vorzugsweise 2 bis 6 Kohlenstoffatomen, oder Alkoxycarbonyl mit vorzugsweise 2 bis 6 Kohlenstoffatomen sein. In manchen Fällen kann X auch NR" mit R" gleich Wasserstoff, Alkyl mit vorzugsweise 1-4 Kohlenstoffatomen oder Aryl mit vorzugsweise 6-12 Kohlenstoffatomen bedeuten.

Bevorzugt für die vorliegende Erfindung sind Kieselsäurepolykondensate mit dem genannten Strukturelement (Ib), worin B die Bedeutung B"-Z- besitzt und Z -O-$R^4$, -S-$R^4$, -NH-$R^4$, -C(O)O-$R^4$, -O-, -S-, -NH- oder -C(O)O- ist. $R^4$ kann dabei die Bedeutung Alkylen, Arylen oder Alkylarylen mit vorzugsweise 1 bis 10 (für ringfreie Gruppen) bzw. 6 bis 14 (für ringhaltige Gruppen) Kohlenstoffatomen haben. B" ist dabei wie B eine geradkettige oder verzweigte organische Gruppe mit mindestens einer (Meth-)Acrylatgruppe und bis vorzugsweise 50 Kohlenstoffatomen.

Die Kieselsäurepoly(teil)kondensate mit dem Strukturelement der Formel (Ib) können gegebenenfalls auch aus einem Gemisch von verschiedenen Silanen der Formel (Ia) abgeleitet sein, in denen die Reste B und/oder B' unterschiedliche Bedeutungen besitzen. In diesen Kondensaten besitzen dann die Reste B und/oder B' keine einheitliche Bedeutung. Da B in diesen Kondensaten einerseits die Bedeutung haben kann, wie sie für B' definiert ist, andererseits aber auch eine davon verschiedene, umfasst die Formel (Ib) Kieselsäurepoly(teil)kondensate, in denen alle Reste B und B' dieselbe Bedeutung haben, Kieselsäurepoly(teil)kondensate, in denen B und B' eine unterschiedliche Bedeutung haben, aber alle Reste B und alle Reste B' jeweils denselben Rest darstellen, und Kieselsäurepoly(teil)kondensate, in denen die Reste B' eine von B verschiedene Bedeutung besitzen und die Reste B und/oder die Reste B' jeweils Mischungen von unterschiedlichen Resten sind. Alternativ oder zusätzlich können diese Kondensate Fremdmetallatome enthalten, die sich in solche Systeme einkondensieren lassen, beispielsweise Bor, Aluminium, Germanium, Zinn, Titan oder Zirkon. Die Metalle, die hierfür geeignet sind, sind dem Fachmann bekannt. Bei fremdmetallhaltigen Kieselsäurepoly(teil)kondensaten handelt es sich dann um Heterokieselsäurepoly(teil)kondensate.

Es ist darauf hinzuweisen, dass die Bestandteile B und B' in den für die vorliegende Erfindung eingesetzten Kieselsäure(teil)kondensaten mit dem Strukturelement (Ib) nicht notwendigerweise im stöchiometrischen Verhältnis zueinander vorhanden sein müssen, wie es sich aus dem Strukturelement selbst ergibt. Wie man auch nachstehend aus der Beschreibung und den Beispielen ersehen kann, kann zum Beispiel der Rest B' unterstöchiometrisch vorhanden sein. In diesen Fällen enthält das Poly(teil)kondensat noch freie (oder durch Umlagerung anderweitig "verkappte" oder "geschützte") Hydroxygruppen, was, wie oben beschrieben, das Viskositätsverhalten der Harze beeinflusst.

Es handelt sich bei den Strukturelementen der vorliegenden Erfindung also um Silane und davon abgeleitete Kieselsäurepoly(teil)kondensate, welche einen teilweise oder vollständig hydrolysierbaren/hydrolysierten und/oder kondensierbaren/kondensierten Silanrest, mindestens eine Urethangruppe und mindestens zwei verzweigt angeordnete, (Meth)Acrylatgruppen enthaltende und damit organisch polymerisierbare Reste, von denen einer über die genannte Urethangruppe an das Siliciumatom gebunden ist, aufweisen. Alle drei Molekülteile können entsprechend dem

Jahrestätigkeitsberichts des Fraunhofer Instituts für Silicatforschung 1992, S.61-72 und Polymer + Materials Research Symposium 1993, Bayreuth, S.14-17, zu Eigenschaftsmodifikationen genutzt werden. Dabei ermöglicht die über die Urethangruppe eingeführte zusätzliche organisch polymerisierbare Gruppe eine zusätzliche Vernetzungsmöglichkeit über die organischen Reste, verglichen mit den Kieselsäurepolykondensaten der DE 44 16 857 C1, wodurch sich festere Polymere erhalten lassen.

Wie weiter unten ausführlich erläutert, kann man die Silane und davon abgeleiteten Kieselsäurepoly(teil)kondensate ausgehend von Silanen erhalten, die einen Rest B sowie eine an einem Linker zwischen diesem Rest B und dem Siliciumatom gebundene Hydroxygruppe aufweisen. Sie sind in der DE 44 16 857 C1 beschrieben. Vergleicht man die Systeme, die man durch direkte Kondensation solcher Silane erhält, mit denjenigen der vorliegenden Erfindung, ist festzustellen, dass die Matrixhydrophilie der erfindungsgemäßen Systeme gegenüber denen der Systeme gemäß DE 44 16 857 C1 herabgesetzt ist, da keine oder, wenn ein Teil der ursprünglichen Hydroxygruppen nicht zur Reaktion gebracht wird, nur eine reduzierte Anzahl von freien OH-Gruppen vorhanden ist/sind, so dass man nassfestere (hydrolytisch resistentere), weniger viskose Harze mit einer geringeren Empfindlichkeit gegenüber Feuchtigkeit erhalten kann. Auf der anderen Seite lässt sich über die Variabilität der Reste B, B', R, $R^1$ und R' eine hohe Variabilität erzielen, die zu besonderen und neuen Eigenschaftskombinationen führt. Ein weiterer Vorteil der vorliegenden Erfindung liegt darin, dass man Kondensate, die frei von den Ausgangsmonomeren sind, und daraus (durch Polymerisation der organisch polymerisierbaren Gruppen) organische Polymere mit guten mechanischen Eigenschaften und geringer Schrumpfung bei Viskositätseigenschaften erhalten kann, die gute Verarbeitungsmöglichkeiten bieten. Solche Polymere werden nachstehend als Polymerisate bezeichnet, wenn sie füllmittelfrei sind, und als Komposite, wenn sie Füllstoffe enthalten.

Über die organisch polymerisierbaren Anteile (vor allem die (Meth-)Acrylatgruppen) der Reste B und B' sind die oben genannten Kieselsäurepolykondensate organisch vernetzbar. Dabei wird wegen des Vorhandenseins von mindestens zwei organisch vernetzbaren Gruppen pro Silanmolekül ein in der Regel von Ausgangsmaterialien freies System erhalten, dessen organischer Anteil zu einer besonders hohen mechanischen Festigkeit sowie überraschenderweise einem verbesserten Schrumpfverhalten mit einer verringerten Schrumpfung führt. Erfindungsgemäß lassen sich diese Systeme in Kombination mit nanopartikulären und ggf. weiteren Füllstoffen als Dentalkomposite mit äußerst vorteilhaften Eigenschaften einsetzen.

Aus den oben genannten Harzen lassen sich demzufolge anorganisch-organische Polymere mit guten mechanischen Eigenschaften (z.B. geringer Schrumpfung) erhalten, die völlig oder im wesentlichen frei von den als Ausgangsmaterialien eingesetzten Monomeren sind, wobei die Harze selbst aufgrund z.B. ihrer günstigen Viskositätseigenschaften gute Verarbeitungsmöglichkeiten erlauben. Diese Polymere werden in Form von Kompositen (d.h. füllmittelhaltig, wobei zumindest ein wesentlicher Anteil des oder der Füllstoffe nano-partikuläre Füllstoffe sind) bereitgestellt und eignen sich aufgrund der günstigen Eigenschaften aus toxikologischer Sicht (z.B. wegen ihrer hohen Biokompatibilität) in Kombination mit hoher Nassfestigkeit und sehr geringer Schrumpfung insbesondere für den Dentalbereich.

Die erfindungsgemäßen Komposite lassen sich vor der Härtung bezüglich der rheologischen Eigenschaften nach Bedarf einstellen. So kann, z.B. durch Zusatz von monomeren Additiven, (z.B. Reaktivverdünnern) die Fließfähigkeit der Materialien erhöht werden, so dass sie sich als Fissurenversiegler oder dgl. eignen. Für andere Anwendungszwecke wie Füllungen, Prothesen werden dagegen höherviskose Materialien benötigt. Dadurch lassen sich die unterschiedlichsten Applikationen im Dentalbereich verwirklichen, z.B. für die oben angegebenen Zwecke. Die Komposite besitzen eine hohe Abrasionsresistenz (bzg. Kauabrieb und Antagonistenabrieb). Bei Bedarf kann ihnen außerdem eine hohe Röntenopazität verliehen werden, indem sie entsprechende röntgenopake Füllstoffpartikel enthalten.

Erhältlich sind die für die vorliegende Erfindung einsetzbaren Verbindungen und (Teil-)Kondensate beispielsweise ausgehend von Verbindungen der Formel II

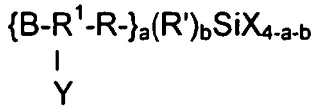

(II)

worin B, $R^1$, R, R', X a und b die für die Formeln (Ia) und (Ib) angegebenen Bedeutungen besitzen und Y OH ist. Die am Siliciumatom befindlichen Substituenten oder Reste R' und X können beliebig ausgewählt werden. In der Literatur über die anorganisch-organischen, Siliciumatome enthaltenden Materialien, z.B. diejenigen, die unter der Bezeichnung "ORMOCERE®" im Handel sind, ist viel über die jeweiligen Eigenschaften geschrieben worden, die die jeweiligen Silanreste dem Kondensat oder organisch polymerisierten Netzwerk verleihen, so dass hier keine detaillierten Erläuterungen nötig sind. Mit X werden hydrolysierbare Reste bezeichnet. Mit diesen Gruppen, die auch als anorganische Netzwerkbildner bezeichnet werden, werden im Zusammenspiel mit ggf. vorhandenen organischen Netzwerkbildnern, hier also insbesondere den organisch polymerisierbaren Gruppen der Reste B und ggf. B', physikalische Eigenschaften des sich bildenden Netzwerks eingestellt wie Härte bzw. Flexibilität oder der thermische Ausdehnungskoeffizient. Die in der

Regel nicht organisch polymerisierbaren Gruppen R' werden als Netzwerkwandler bezeichnet; mit ihrer Wahl lassen sich ebenfalls eine Reihe von Eigenschaften beeinflussen. Dem Fachmann ist dementsprechend bekannt, welche Bedeutung X annehmen kann. Vorzugsweise ist X eine $C_1$-$C_{10}$-Alkoxygruppe, stärker bevorzugt eine $C_1$-$C_4$-Alkoxygruppe und ganz besonders bevorzugt Methoxy oder Ethoxy. X kann aber auch je nach Bedarf ein Halogenid wie Cl, Wasserstoff, Hydroxy, Acyloxy mit vorzugsweise 2 bis 5 Kohlenstoffatomen, Alkylcarbonyl mit vorzugsweise 2 bis 6 Kohlenstoffatomen, Alkoxycarbonyl mit vorzugsweise 2 bis 6 Kohlenstoffatomen, ggf. auch NR" mit R" gleich Wasserstoff, Alkyl mit vorzugsweise 1-4 Kohlenstoffatomen oder Aryl mit vorzugsweise 6-12 Kohlenstoffatomen, oder eine andere geeignete Abgangsgruppe sein.

Verbindungen der Formel (II) sind bekannt. So lassen sich beispielsweise gemäß der DE 44 16 857 C1 Verbindungen der Formel (II) herstellen, in denen B die Bedeutung B"-Z- besitzt, wobei auch B" die Bedeutung einer geradkettigen oder verzweigten organischen Gruppe mit mindestens einer (Meth)Acrylatgruppe und bis vorzugsweise 50 Kohlenstoffatomen mit den für B beschriebenen bevorzugten Ausgestaltungen besitzt. Setzt man zum Beispiel Epoxid-Silane mit Verbindungen B"(AH) um, in denen AH eine Hydroxy-, eine Mercapto- oder eine Aminogruppe oder ein Carbonsäurerest ist, erhält man Produkte, in denen Y -OH bedeutet und Z -O-R", -S-R", -NH-R", - C(O)O-R", -O-, -S-, -NH- oder -C(O)O- ist. R" besitzt dabei die oben angegebene Bedeutung. Die Umsetzung erfolgt meist in Gegenwart eines geeigneten Katalysators, z.B. tertiärer Amine wie Triethylamin oder Phosphine wie Triphenylphosphin, und ggf. bei erhöhten Temperaturen.

Bei den voranstehend beschriebenen Umsetzungen zur Herstellung der Verbindungen der Formel (II) können je nach tatsächlich eingesetzten Ausgangsmaterialien Isomere dieser Verbindungen entstehen. Dies ist insbesondere dann in erheblichem Maße der Fall, wenn die Reste X Alkoxygruppen, vor allem Methoxy- oder Ethoxygruppen sind. Da in solchen Isomeren teilweise die Gruppe Y in die Isomerisierungs-/Umesterungsreaktion involviert ist, ist sie in diesen Produkten teilweise nicht mehr frei. Es hat sich aber herausgestellt, das diese Nebenprodukte genauso gut für die Herstellung der den Kompositen der Erfindung zugrundeliegenden Kieselsäurepoly(teil)kondensate herangezogen werden können, wie die Verbindungen der Formel (II) selbst, wobei eine Trennung der verschiedenen Produkte gar nicht notwendig ist. Es ist statt dessen ausreichend, die für die Herstellung der Verbindungen mit der Formel (II) beschriebenen Ausgangsmaterialien miteinander in der angegebenen Weise umzusetzen und anschließend einer Hydrolyse zu unterwerfen. Überraschenderweise wird dabei die Gruppe Y wieder frei, während die Rückbildung von SiOH-Gruppen unterschiedlich stark ausfällt und im Wesentlichen unterdrückt werden kann. Deshalb erhält man in der Regel ein Kondensat mit einem Si-O-Si-Netzwerk.

Die Verbindungen der Formel (II) bzw. ihre Kondensationsprodukte mit wieder freigesetzter Gruppe Y werden bei Bedarf aufgearbeitet (z.B. getrennt, gewaschen, isoliert) und/oder, wenn erforderlich, getrocknet. Insbesondere sollte dabei ggf. darauf geachtet werden, dass keine oder möglichst wenig H-aktive Verunreinigungen im Reaktionsgemisch vorhanden sind, um in der nachstehend beschriebenen Umsetzung Nebenreaktionen mit dem Isocyanat zu vermeiden. Sodann werden sie mit einem mindestens eine (Meth-)Acrylatgruppe aufweisenden Isocyanat umgesetzt, wobei ein Produkt entsteht, in dem eine zweite (Meth-)Acrylatgruppe über eine Urethangruppe -NH-C(O)O- gebunden vorliegt.

Wenn monomere Verbindungen der Formel (II) umgesetzt wurden, wird man in der Regel Silane der Formel (Ia) erhalten. Diese können anschließend einer hydrolytischen Kondensation unterworfen werden, um zu Kondensaten der Formel (Ib) zu gelangen.

Die Herstellung der für den voranstehenden Weg zur Herstellung der Harze erläuterten Silan-Verbindungen kann auf verschiedene Arten erfolgen. Einige Verfahrensvarianten seien nachstehend prinzipiell beschrieben.

Im ersten Schritt der Herstellung wird in einer ersten Ausgestaltung der-Erfindung eine Verbindung B"(COOH), worin B" die obige Bedeutung besitzt, mit einem einen Oxiranring enthaltenden Silan $[CH_2$-$CH(O)]$-R-$Si(X)_3$ umgesetzt, in dem R und X die für die Formeln (Ia), (Ib) und (II) angegebenen Bedeutungen besitzen. Vorzugsweise ist X eine Methoxy, Ethoxy-, Propoxy- oder Butoxygruppe. R kann beispielsweise $-CH_2$-O-$(CH_2)_3$ sein. Die Umsetzung erfolgt vorzugsweise in Gegenwart eines Katalysators wie oben erläutert und bei erhöhten Temperaturen. Wie voranstehend beschrieben, entstehen bei dieser Umsetzung je nach Ausgangsmaterial und Reaktionsbedingungen nicht nur Verbindungen der Formel (II), sondern auch oder sogar ausschließlich Kondensationsprodukte (Umesterungsprodukte), beispielsweise durch Verlust eines Moleküls Alkohol unter Ausbildung von Brückenbindungen zwischen dem Sauerstoffatom der sich bildenden Hydroxygruppe (der Gruppe Y in Formel (II)) und einem Siliciumatom, gemäß dem folgenden Schema, das anhand des Beispiels einer Umsetzung von Methacrylsäure mit 3-Glycidyloxypropyltrimethoxysilan ("Glymo") gezeigt sei:

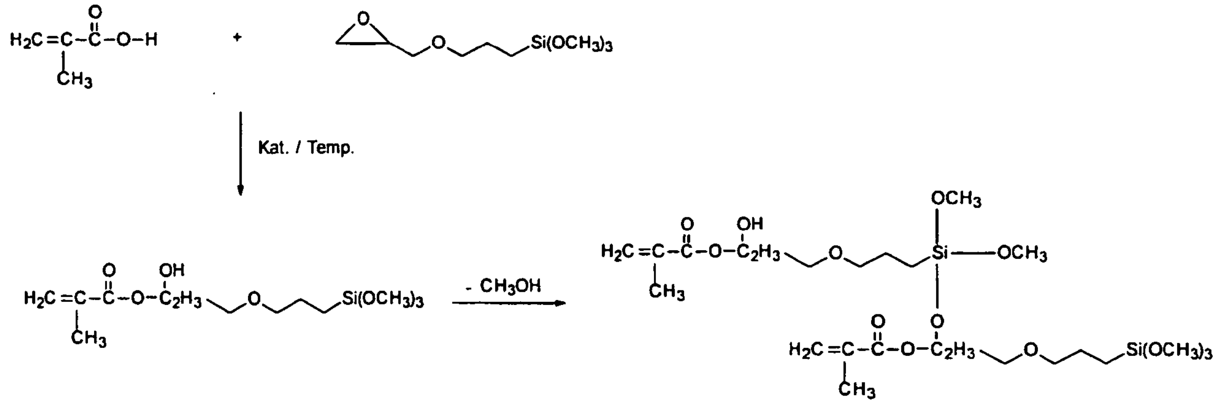


Das obige Schema zeigt ein intermolekular umgelagertes Produkt. Es sollte klar sein, dass natürlich auch die Produkte von intramolekularen Kondensations- bzw. Umesterungsreaktionen eingesetzt werden könnten.

Das Produkt bzw. die Produktmischung kann, sofern die OH-Gruppen nicht vollständig hydrolysiert sind, einer Hydrolyse unterworfen werden, die unter anderem eine Kondensation der Silanreste bewirkt. Überraschenderweise entstehen dabei dann, wenn für X eine Alkoxygruppe gewählt wird, nicht unbedingt nennenswerte Mengen an freien Hydroxygruppen an den Siliciumatomen, während die Hydroxygruppe an der Position Y zurückgebildet wird. Wie bereits voranstehend im allgemeineren erläutert, lässt sich nämlich die Bildung freier Hydroxygruppen am Siliciumatom einstellen und bei Bedarf im Wesentlichen unterdrücken. Man kann demzufolge ein Kieselsäurepolykondensat oder -Teilkondensat erhalten, das zu sehr großen, ggf. überwiegenden Teilen oder sogar vollständig das folgende Strukturelement (III)

$$\begin{array}{l}\{B\text{-}R^1\text{-}R\text{-}\}_a(R')_bSi(O\text{-})_{4-a-b}\\ \quad |\\ \quad Y\end{array}\qquad \text{(III)}$$

aufweist, worin die angegebenen Reste und Indices die voranstehend erläuterte Bedeutung besitzen.

Die vorstehend beschriebene Hydrolyse wird jedoch häufig nicht notwendig sein. Denn die Silylierung der Hydroxygruppe kann diese ggf. "schützen". Wenn im Reaktionsgemisch teils freie, teils geschützte OH-Gruppen vorliegen, lässt sich daraus leicht ein Kondensat der Formel (Ib) erzeugen, in dem die Reste B' in Relation zu B im Unterschuss vorliegen. Dass dies wünschenswert sein kann, ist voranstehend beschrieben.

In einem alternativen Herstellungsweg für das als Bestandteil der erfindungsgemäßen Dentalmaterialien einsetzbare Heteropolysiloxan wird der erste Schritt durch die Umsetzung einer Verbindung B"(OH), worin B" die obige Bedeutung besitzt, mit einem einen Oxiranring enthaltenden Silan $[CH_2\text{-}CH(O)]\text{-}R\text{-}Si(X)_3$ umgesetzt, in dem R und X die für die Formeln (Ia), (Ib) und (II) angegebenen Bedeutungen besitzen. Vorzugsweise ist X eine Methoxy, Ethoxy-, Propoxy- oder Butoxygruppe. R kann beispielsweise $-CH_2\text{-}O\text{-}(CH_2)_3$ sein. Die Umsetzung erfolgt vorzugsweise in Gegenwart eines Katalysators wie oben erläutert und bei erhöhten Temperaturen gemäß dem folgenden Schema, das anhand des Beispiels einer Umsetzung von HEMA (Hydroxyethylmethacrylat) mit 3-Glycidyloxypropyltrimethoxysilan ("Glymo") gezeigt sei:

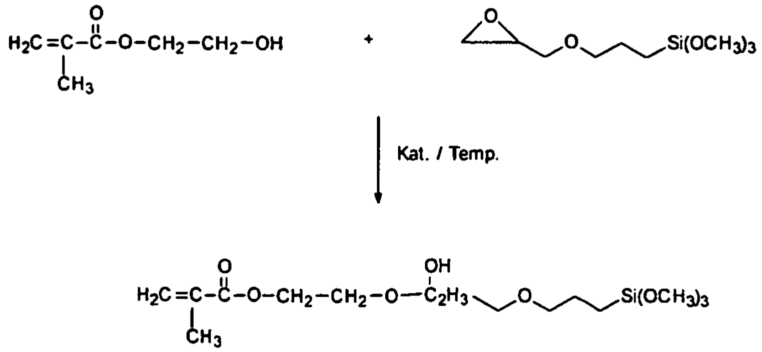


Wie voranstehend beschrieben, können auch bei dieser Umsetzung je nach Ausgangsmaterial nicht nur eine Verbindung der Formel (II), sondern auch oder sogar ausschließlich Kondensationsprodukte entstehen, beispielsweise durch Verlust eines Moleküls Alkohol unter Ausbildung von Brückenbindungen zwischen dem Sauerstoffatom der sich bildenden Hydroxygruppe (der Gruppe Y in Formel (II)) und einem Siliciumatom. Prinzipiell sind für diese Reaktion dementsprechend die folgenden Umesterungen möglich:

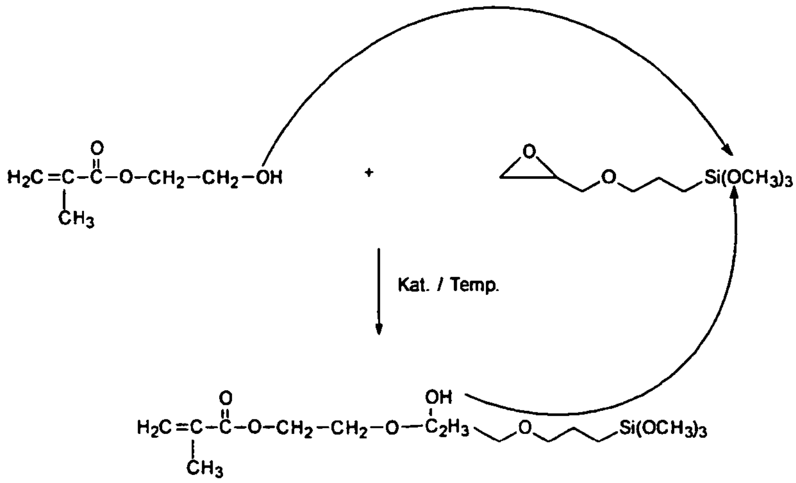


Der nächste Schritt der Herstellung der Kondensate kann wie nachstehend beschrieben erfolgen: Das Produkt des ersten Schrittes wird mit einer Verbindung B'NCO umgesetzt, worin B' die voranstehend genannte Bedeutung besitzt. Dabei entsteht eine Verbindung mit der Formel (Ia) oder ein Kondensat mit dem Strukturelement (Ib).

Der zweistufige Syntheseweg der für die Zwecke der vorliegenden Erfindung einsetzbaren Silanharze sei nachstehend anhand einiger Schemata nochmals verdeutlicht:

1. Hydroxyfunktionalisiertes Dimethacrylat aus DE 4416857

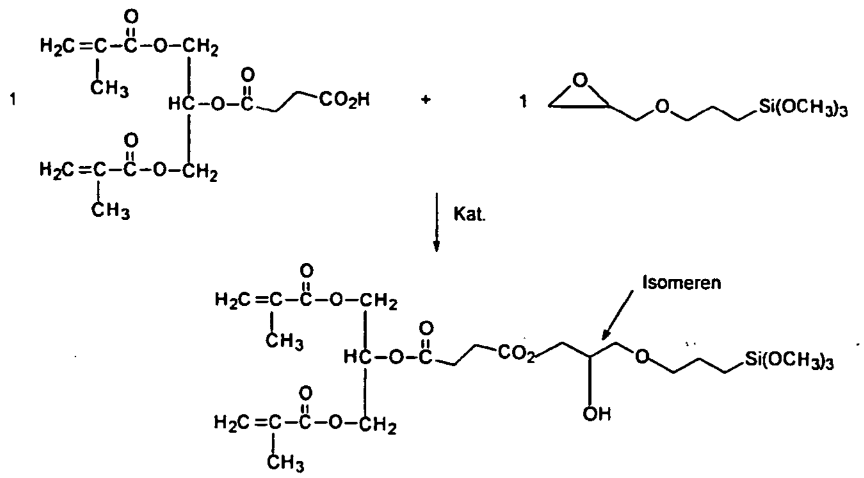


1a. Umsetzung obiger Hydroxyfunktionalisierter Dimethacrylate

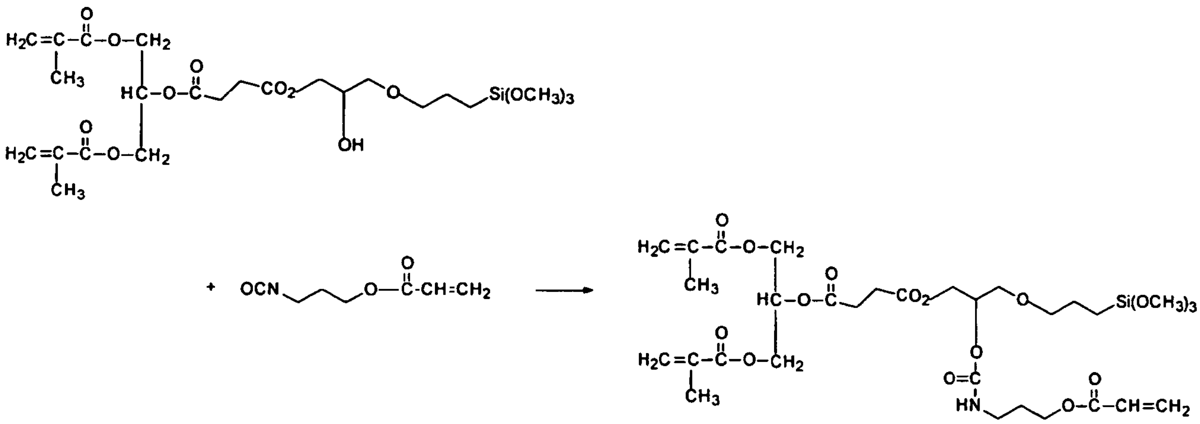

2. Hydroxflunktionalisiertes Dimethacrylat aus DE 4416857

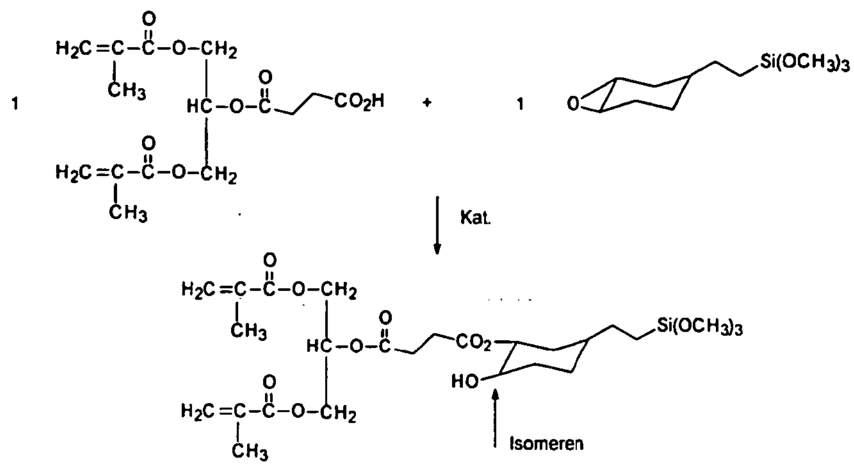


2a. Umsetzung obiger Hydroxyfunktionalisierter Dimethacrylate

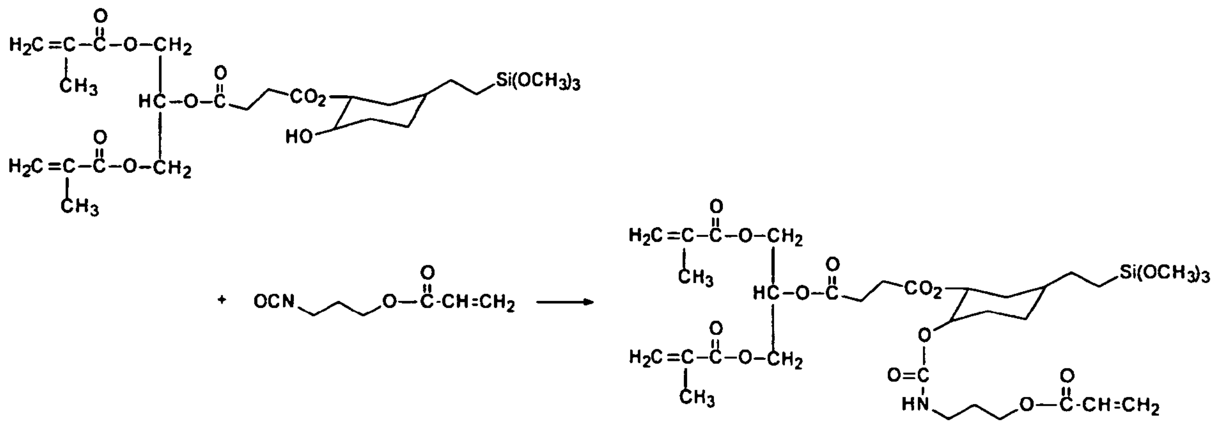

Nachstehend sei die Herstellung von Verbindungen der Formel (Ia) außerdem anhand einiger ausgewählter Umsetzungen illustriert:

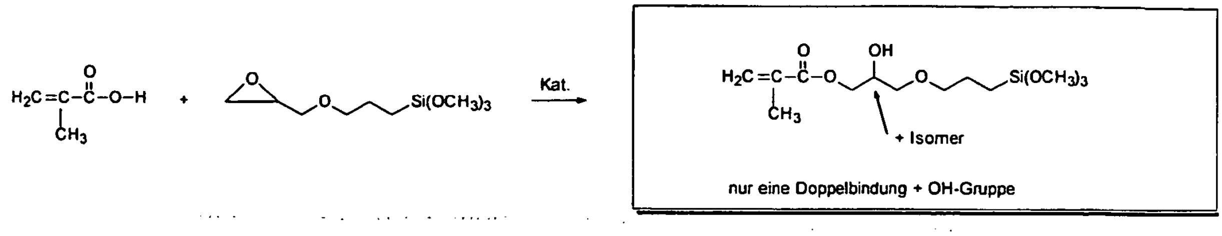


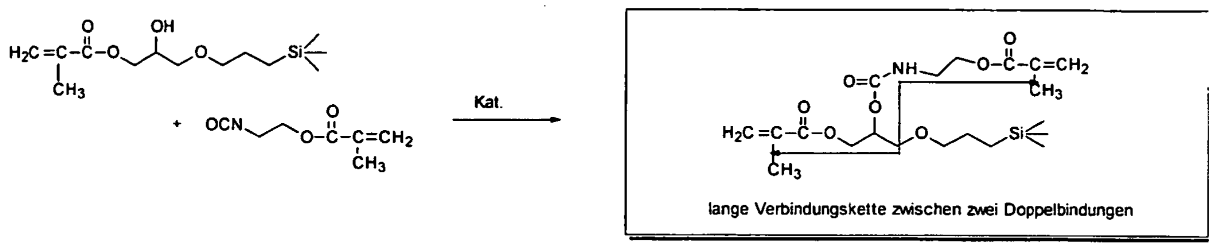


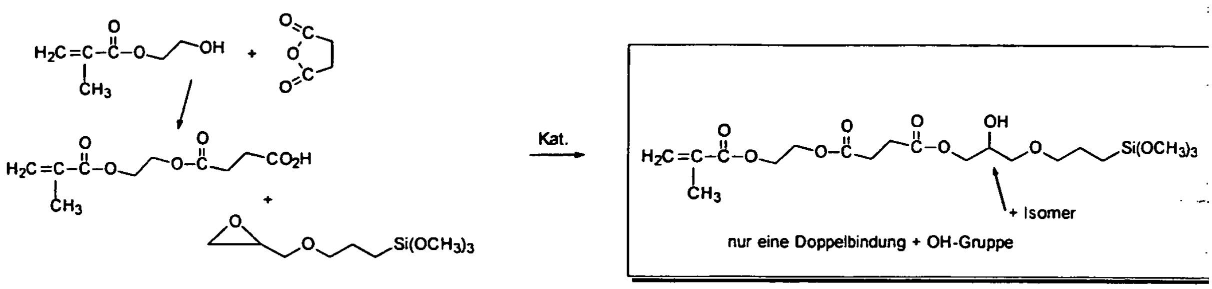

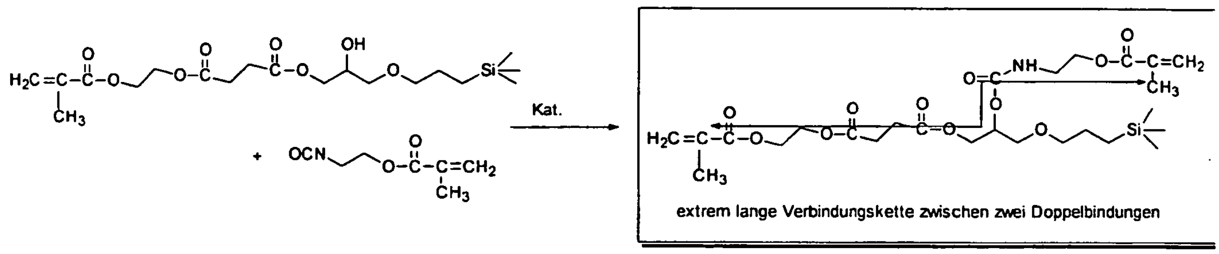


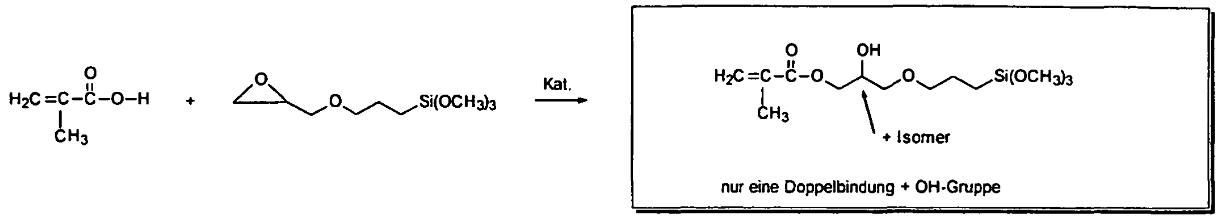


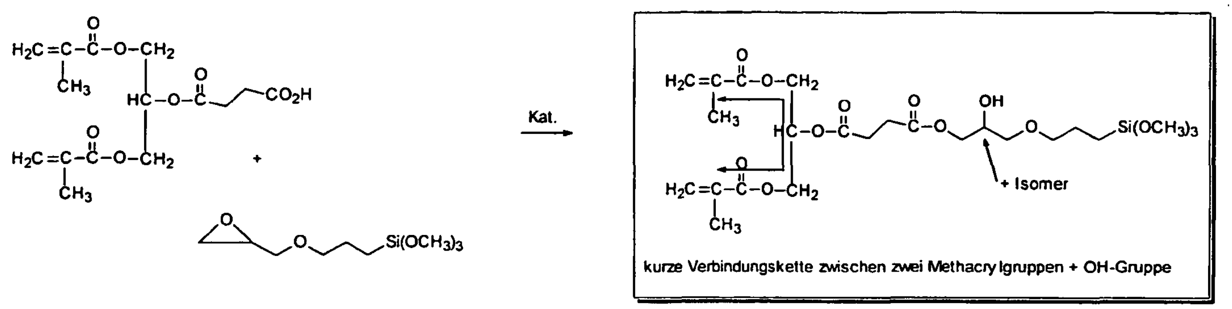


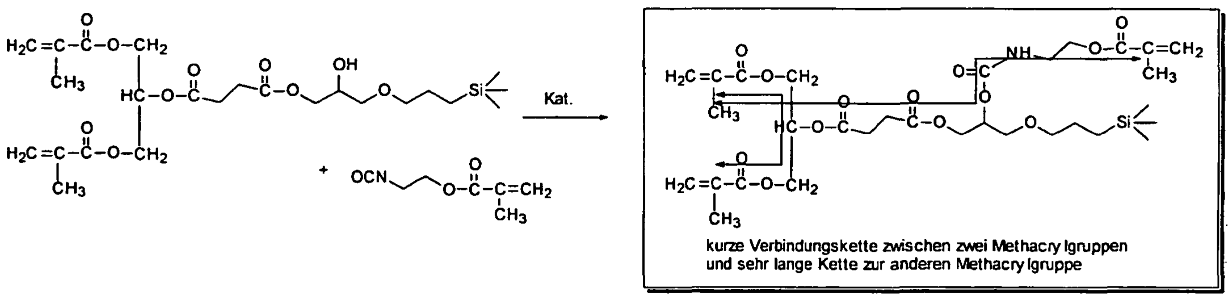


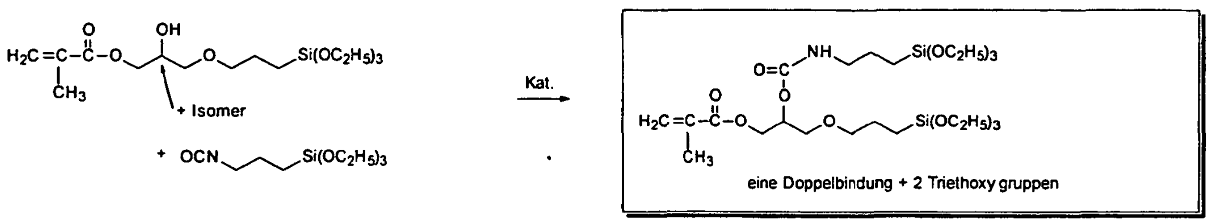


Die voranstehenden Schemata zeigen, dass sich gemäß der DE 103 49 766.8 im Rahmen einer Isocyanataddition sehr variabel gebaute Silane erzeugen lassen. So können aus dem Produkt der Umsetzung von (Meth-)Acrylsäure mit Glymo, das aus der DE 44 16 857 C1 bekannt ist, Silane der Formel (Ia) hergestellt werden, die je nach eingesetztem Reaktionspartner relativ lange oder relativ kurze Verbindungsketten zwischen den Doppelbindungen der Reste B und B' aufweisen (siehe obere Hälfte der ersten Schemaseite). Aus demselben Patent ist das Produkt der Umsetzung von Hydroxyethylmethacrylat (HEMA), Bernsteinsäureanhydrid und Glymo bekannt. Dieses Produkt enthält einen einzigen

Methacrylsäurerest sowie eine relativ weit entfernt davon stehende Hydroxygruppe. Daraus lassen sich Verbindungen mit zwei Methacrylatgruppen herstellen, zwischen denen sehr lange bzw. extrem lange Verbindungsketten angeordnet sind. In der Mitte der zweiten Schemaseite ist die Herstellung von Verbindungen mit zwei recht nahe beieinanderstehenden, doppelbindungshaltigen Gruppen (hier Methacrylatgruppen) sowie einer dritten, von diesen weiter entfernt angeordneten doppelbindungshaltigen Gruppe (hier ebenfalls eine Methacrylatgruppe, diese könnte jedoch auch eine andere doppelbindungshaltige Gruppe sein) anhand eines Beispiels gezeigt.

Für die Zwecke der vorliegenden Erfindung werden Silane mit der Formel (Ia) bzw. noch nicht vollständig durchkondensierte Kieselsäurepoly(teil)kondensate mit Strukturelementen gemäß Formel (Ib) allein oder ggf. auch mit weiteren Silanen und/oder Kieselsäure(teil)kondensaten teilweise, weiter oder vollständig hydrolysiert und kondensiert. Hierfür eignen sich zum einen Silane und (Teil-)Vorkondensate daraus, die cokondensierbar, aber nicht copolymerisierbar sind, oder solche, die ebenfalls über polymerisierbare Gruppen verfügen. Selbstverständlich lassen sich die weiteren Komponenten auch bereits in einem früheren Stadium einarbeiten, wenn sie nicht mit Isocyanaten unerwünschte Nebenreaktionen eingehen können. Dabei entstehen Kondensate mit ausschließlich den erfindungsgemäßen Struktureinheiten der Formel (Ib) oder anorganische Netzwerke mit Si-O-Si-Einheiten, die diese Struktureinheiten in Kombination mit anderen Einheiten enthalten. Cokondensierbare bzw. copolymerisierbare Verbindungen bzw. (Teil-)Vorkondensate können bevorzugt in einem molaren Verhältnis von bis zu 80%, bezogen auf die Monomereinheiten (Silylreste bzw. polymerisierbare Verbindungen), aus denen das resultierende Harz oder Polymer aufgebaut ist, zugesetzt werden. Besonders bevorzugt ist ein Zusatz in einem molaren Verhältnis von bis zu ca. 20%.

Den Silanen der Formel (Ia) sowie noch nicht vollständig durchkondensierten Kieselsäurepoly(teil)kondensaten mit Strukturelementen der Formel (Ib) können statt dessen oder zusätzlich copolymerisierbare Komponenten zugesetzt werden, bei denen es sich z.B. um radikalisch und/oder ionisch und/oder kovalent-nucleophil polymerisierbare Verbindungen handelt. Radikalisch polymerisierbare Verbindungen, die zugesetzt werden können, sind z.B. solche mit C=C-Doppelbindungen, wie z.B. Acrylate oder Methacrylate, wobei die Polymerisation über die C=C-Doppelbindungen erfolgt. Ionisch polymerisierbare Verbindungen, die zugesetzt werden können, enthalten z.B. Ringsysteme, die kationisch ringöffnend polymerisierbar sind, wie z.B. Spiroorthoester, Spiroorthocarbonate, bicyclische Spiroorthoester, Mono- oder Oligoepoxide oder Spirosilane, wie sie z.B. aus der DE 41 25 201 C1 bekannt sind. Es können aber auch Verbindungen zugesetzt werden, die sowohl ionisch als auch radikalisch polymerisierbar sind, wie z.B. Methacryloyl-Spiroorthoester. Diese sind radikalisch über die C=C-Doppelbindung und kationisch unter Ringöffnung polymerisierbar. Die Herstellung dieser Systeme ist z.B. im Journal f. prakt. Chemie, Band 330, Heft 2, 1988, S. 316-318 beschrieben. Ferner ist es z.B. möglich, andere bekannte Silan-gebundene cyclische Systeme zuzusetzen, die mit einpolymerisiert werden können. Solche Systeme sind z.B. solche, die Epoxide enthalten. Solche Systeme sind bei der Herstellung der Spiro-Silane in der DE 41 25 201 C1 beschrieben. Die vorgenannten Komponenten werden bei der Polymerisation der Harze über ihre organisch polymerisierbaren Gruppen einpolymerisiert, so dass man ein Copolymerisat aus erfindungsgemäßen Silanen und Copolymeren erhalten kann, dessen Silangruppen untereinander oder mit weiteren Gruppen hydrolytisch (teil-)-kondensiert vorliegen. Die genannten Komponenten können bevorzugt in einem molaren Verhältnis von bis zu 20%, bezogen auf die Monomereinheiten (Silylreste bzw. polymerisierbare Verbindungen), aus denen das resultierende Harz oder Polymer aufgebaut ist, zugesetzt werden. Besonders bevorzugt ist es jedoch, die Komposite der vorliegenden Erfindung und damit auch die zugrunde liegenden organisch polymerisierbaren Harze monomerfrei zu halten.

Wie oben bereits angedeutet, kann man in einem alternativen Syntheseweg zur Herstellung der für die Erfindung geeigneten Harzsysteme von den Verbindungen der Formel II ausgehen, die erst einmal hydrolytisch kondensiert werden, bevor die Umsetzung mit einem (meth-)acrylatgruppenhaltigen Isocyanat erfolgt. Bis auf diese Umkehrung gelten alle vorgenannten Erläuterungen auch für diesen Weg der Harzsystem-Herstellung.

Die für die Zwecke der vorliegenden Erfindung einsetzbaren Kieselsäurepoly(teil)kondensate mit Strukturelementen der Formel (Ib) besitzen dann, wenn Y vollständig oder zu hohen Anteilen umgesetzt ist, eine niedrige Matrixhydrophilie und nehmen dementsprechend in feuchter/nasser Umgebung nur wenig Wasser auf. Ihre Nassfestigkeit ist verbessert. Die Reste B und B' lassen sich allein oder in Mischungen und/oder Cokondensaten mit weiteren Bestandteilen wie den oben erwähnten in organische Polymerstrukturen einbauen, oder sie können über diese Gruppen als solche vernetzt werden. Aufgrund der zusätzlichen organischen, vernetzbaren Gruppe oder der zusätzlichen Silylgruppe, die B' trägt, lässt sich eine generelle Steigerung der Festigkeit der vernetzten Produkte erzielen. Insbesondere kann man niedriger oder auch hoch gefüllte Komposite erhalten, die aus Harzen mit relativ geringer Viskosität erhalten wurden und die eine sehr geringe Schrumpfung aufweisen. Ihre Eignung als Dentalmassen ist besonders hervorzuheben, insbesondere in solchen Ausgestaltungen, die monomerfrei und damit toxikologisch/allergologisch unbedenklich sind, und sie besitzen in der Regel darüber hinaus eine hohe Nassfestigkeit sowie andere, bereits voranstehend genannte vorteilhafte Eigenschaften.

Zum Erhalt des Dentalkomposits wird das Kieselsäurepoly(teil)kondensat mit Strukturelementen gemäß Formel (Ib) vor der organischen Aushärtung mit einem oder mehreren Zusatz- und/oder Füllstoffen gemischt.

Wesentlicher Bestandteil in dieser Hinsicht sind nanopartikuläre Füllstoffe oder eine Kombination solcher Füllstoffe verschiedener Größe oder verschiedener Zusammensetzung, ggf. in Kombination mit weiteren, bekannten Füllstoffen wie partikulären Dentalgläsern, z.B. Ba-Sr-Aluminiumborosilikaten. Unter "nanopartikulär" ist dabei zu verstehen, dass die Füllstoffe einen Durchmesser oder ihren größten Durchmesser im Bereich von unter 1000 nm aufweisen. Für den Fall, dass die Füllstoffe eine relativ breite Korngrößenverteilung aufweisen, sollen wenigstens 90% der Masse des Füllstoffs unterhalb dieser Grenze liegen. Umfasst vom Ausdruck "nanopartikulärer Füllstoff' sollen erfindungsgemäß dabei auch subnanopartikuläre Füllstoffe sein, deren Größe sich hinunter bis zu der Größe so genannter Cluster erstrecken kann. Bevorzugt werden nanopartikuläre Füllstoffe mit annähernd sphärischer Form eingesetzt. Stärker bevorzugt werden Füllstoffe mit Durchmessern im Bereich von 10 bis 400 nm, noch stärker bevorzugt im Bereich von 10 bis 100 nm eingesetzt. Bevorzugt ist es außerdem, jeden einzelnen Füllstoff mit einer engen Korngrößenverteilung einzusetzen.

Die nanopartikulären Füllstoffe können einzeln, d.h. in isolierter Form, als Agglomerate, Aggregate oder Polymerisate vorliegen. Bevorzugt ist es, dass sie in nichtagglomerierter, nichtaggregierter Form verwendet werden.

Die Materialien für die genannten Füllstoffe sind nicht kritisch und werden je nach Bedarf ausgewählt. Gut geeignet sind z.B. solche, wie sie in den Füllstoffen der Druckschriften DE 196 43 781, DE 100 41 038 oder DE 100 18 405 eingesetzt werden. Sehr gut geeignet sind $SiO_2$-Partikel, die man beispielsweise nach bekannten Sol-Gel-Verfahren erhalten kann und die dann eine sehr enge Durchmesserverteilung aufweisen können. Diese oder auch anders zusammengesetzte Nanopartikel können auf ihrer Oberfläche modifiziert, z.B. silanisiert sein, um ihre Oberflächeneigenschaften an diejenigen der Matrix anzupassen.

Die nanopartikulären Füllstoffe können allein oder in Kombination mit anderen Füllstoffen für die Zwecke der vorliegenden Erfindung eingesetzt werden. Verwendbar als weitere Füllstoffe sind z.B. Makrofüller (z.B. aus Glas, Keramik oder Quartz, Teilchengrößen zwischen 2 bis 50 $\mu$m), homogene Mikrofüller (beispielsweise aus pyrogener Kieselsäure, Teilchengrößen ca. 0,02 bis 0,06, vorzugsweise ca. 0,04 $\mu$m), inhomogene Mikrofüller (Beispiel: ein Teil der pyrogenen Kieselsäure liegt als Splitterpolymerisat vor), Hybridfüller (Mischungen von Makro- und Mikrofüllern) oder Feinsthybridfüller (z.B. Mischungen aus Aerosil und Ba- oder Sr-Glas mit Teilchengrößen im Bereich von etwa 1 bis 5 $\mu$m). Sehr gut geeignet für die vorliegende Erfindung sind beispielsweise Dentalgläser mit Teilchendurchmessern von ca. 0,4 bis 20 $\mu$m, bevorzugt von ca. 1-5 $\mu$m.

Das Verhältnis der Füllstoffe untereinander kann beliebig gewählt werden. Günstig sind Gewichtsanteile des nanopartikulären Füllstoffs von etwa 5 bis etwa 60 Gew.-%, bezogen auf das Gesamtgewicht an Füllstoff im Komposit. Besonders günstig sind Anteile von über 5 bis 30 Gew.-%. In solchen Ausgestaltungen können die nanopartikulären Füllstoffe in den Hohlräumen oder Lücken zwischen engen, möglicherweise sogar annähernd dichtesten (Kugel-)Pakkungen von größeren Füllstoffteilchen liegen, insbesondere, wenn die größeren Füllstoffteilen eine annähernd sphärische Gestalt besitzen. Es hat sich herausgestellt, dass beim Einsatz von Anteilen in diesem Bereich besonders hoch gefüllte Komposite erhältlich sind, die eine besonders geringe Schrumpfung und eine besonders hohe Abrasionsfestigkeit aufweisen.

Der Füllstoff kann je nach gewünschtem Anwendungsbereich in sehr unterschiedlichen Gesamtmengen zugesetzt werden. So kann er z.B. einerseits in einem Anteil von 50 Gew.-% des Komposits oder sogar deutlich darüber und insbesondere in einem Anteil von 70 bis 90 Gew.-% des Komposits vorliegen, wenn höher- oder hochgefüllte Komposite benötigt werden, z.B. für Füllungen oder dergleichen, andererseits für niedriger bzw. niedrig gefüllte Zusammensetzungen, z.B. Fissurenversiegler, Zahnhalsbeschichter, in einem Anteil von weniger als 50 Gew.%, z.B. 1-50 Gew.-% und bevorzugt von ca. 1 bis 20 Gew.-% vorliegen.

Im Hinblick auf die Erzielung hoher Füllstoffgehalte und einer guten Verarbeitbarkeit (z.B. Reduzierung der Viskosität) ist ggf. auch der Einsatz von Füllstoff-Präpolymerisaten, d.h. von mit (der erfindungsgemäßen oder einer anderen, z.B. rein organischen) Polymermatrix überzogenen, nanopartikulären und/oder konventionellen Füllstoffen sinnvoll, die in Form von ausgehärteten Komposit-Kugeln oder -Splittern vorliegen.

Je nach dem vorgesehenen speziellen Verwendungszweck können dem Komposit außerdem geeignete Additive wie Initiatoren, Färbemittel (Farbstoffe oder Pigmente), Oxidationsinhibitoren, Polymerisationsinhibitoren (für die Vermeidung einer vorzeitigen Polymerisation), Verlaufsmittel, UV-Absorber, Stabilisatoren, mikrobiozide Wirkstoffe oder dergleichen zugesetzt werden, wie es dem Fachmann bekannt ist. Beispiele für Polymerisationsinitiatoren sind Initiatoren für die radikalische Polymerisation, und zwar für die thermische Härtung wie Peroxide (z.B. Dibenzoylperoxid) oder Photoinitiatoren wie Benzophenon, Campherchinon oder Kombinationen von $\alpha$-Diketonen mit Aminen als Reduktionsmittel, wie beispielsweise aus der DE 199 03 177 C2 bekannt. Für die duale Aushärtung von radikalisch und kationisch polymerisierbaren Systemen können insbesondere Diaryliodonium- oder Triarylsulfoniumsalze zugesetzt werden, für die die vorgenannte Druckschrift ebenfalls Beispiele angibt.

Die resultierenden plastisch verarbeitbaren Komposite zeichnen sich also dadurch aus, dass sie sehr variable Füllstoffgehalte aufweisen können und gleichzeitig ausgezeichnet verarbeitbar sind. Das ermöglicht in vorteilhafter Weise ihren Einsatz auf dem Gebiet der Dentalrestauration/Prophylaxe (z.B. für Füllungen, als Fissurenversieglern und dgl.). Die gehärteten Komposite zeigen unter anderem eine hohe Festigkeit und einen E-Modul, der an den jeweiligen Verwendungszweck angepasst bzw. angenähert werden kann (so besitzt Dentin einen E-Modul von ca. 18 GPa bzw. in Flüssigkeit ca. 10 GPa). Gleiches gilt für den thermischen Ausdehnungskoeffizienten $\alpha$, der für Dentin bei ca. $8x10^{-6}$

$K^{-1}$ liegt. Ferner besitzen sie eine drastisch erhöhte Abrasionsresistenz, was den Kauabrieb sehr gering hält. Außerdem schrumpfen sie, wie bereits oben ausgeführt, beim Härten (der organischen Vernetzung) überraschenderweise nur sehr gering (siehe Beispiele). Darüber hinaus sind sie, wenn gefordert, von hoher Röntgenopazität, was sich vor allem durch Einarbeitung entsprechender röntgenopaker Füllstoffe, insbesondere der erwähnten Dentalgläser und nanopartikulären Füllstoffe bewirken lässt, unter denen viele eine hohe Röntgenopazität aufweisen. Details hierzu sind dem Fachmann bekannt. So bewirkt beispielsweise ein Austausch von $SiO_2$-Partikeln gegen $SiO_2/SnO_2$-Partikel eine deutliche Erhöhung der Röntgenopazität. Und schließlich besitzen die erfindungsgemäßen Komposite eine ausgezeichnete Ästhetik, weil das Grundharz in einer großen Zahl von Ausgestaltungen im wesentlichen farblos bzw. transluzent ist, was sich ohne weiteres insbesondere aufgrund abgestimmter/angenäherter Brechzahlen von Harz und Füllstoffpartikeln und aufgrund der Partikelverteilung und deren Größe ergibt.

Das gefüllte Dentalkomposit (d.h. das organisch noch nicht vernetzte, gefüllte Harz) kann, nachdem es für den vorgesehenen Zweck angewendet, z.B. in einer Zahnkavität appliziert oder in eine Form gefüllt wurde, die einer späteren Prothese entspricht, in geeigneter Weise organisch vernetzt und damit ausgehärtet werden. Hierfür kommt vor allem eine organische Polymerisation der (Meth-)Acrylatgruppen in Frage. Diese ist eine radikalische Polymerisation, die üblicherweise unter Zusatz von Radikalstartern wie den oben erwähnten und ggf. bekannten Aktivatoren unter Belichtung mit z. B. sichtbarem Licht (Blaulicht; Dentalstrahler) d. h. photochemisch, thermisch oder redoxinduziert erfolgt. Je nach weiteren Zusätzen zum Harz, wie sie oben genannt sind, können zusätzliche photochemisch, thermisch oder chemisch (zum Beispiel über selbsthärtende Zweikomponentenreaktionen, anaerob, redox, ionisch, kovalent-nucleophil) induzierte Umsetzungen stattfinden, durch die die Harzmatrix noch engmaschiger vernetzt werden kann. So ist beispielsweise die Kombination von Selbsthärtung mit z.B. photoinduzierter bzw. thermischer Härtung möglich.

Die erfindungsgemäßen Komposite zeigen aufgrund der wechselseitig aufeinander abstimmbaren Komponenten nicht nur hervorragenden Daten bzgl. einzelner Eigenschaften, sondern auch ein ausgezeichnetes Gesamteigenschaftsprofil und damit deutliche Vorteile gegenüber klassischen, auf methacrylathaltigen Monomeren aufgebauten Kompositen (s. z. B. Komposit 1 und 2 in der Tabelle des Beispiels 3 unten) Denn die Matrix lässt sich bezüglich der Rheologie und Festigkeit optimal auf den Anwendungsbereich einstellen, während sich die nanopartikulären Füllstoffe bezüglich ihres Durchmessers, ihrer Röntgenabsorption, ihrer Oberflächenfunktionalität und dergleichen optimal auswählen lassen. Es resultieren Komposite mit Füllstoffgehalten, die höher sein können als in "klassischen" Materialien und dabei insbesondere einen sehr hohen Volumenanteil im Komposit einnehmen, mit extrem geringer Schrumpfung, mit (Abrasions-)Festigkeiten, die denen der benachbarten Naturmaterialien entspricht, und zwar in der Regel, ohne dass sie zur Verarbeitung mit Verdünnern (also Monomeren) versetzt werden müssten.

Nachstehend soll die Erfindung anhand von Ausführungsbeispielen näher erläutert werden.

**Beispiel 1** (Stand der Technik)

Dieses Beispiel erläutert die Herstellung eines monomerfreien Harzsystems, das freie OH-Gruppen enthält, ausgehend von einer Verbindung der Formel (II) mit b gleich 0; letztere ist auch der DE 103 48 766.8 zu entnehmen:

1. Stufe: Umsetzung von 3-Glycidyloxypropyltrimethoxysilan (Glymo) mit Methacrylsäure (MAS)

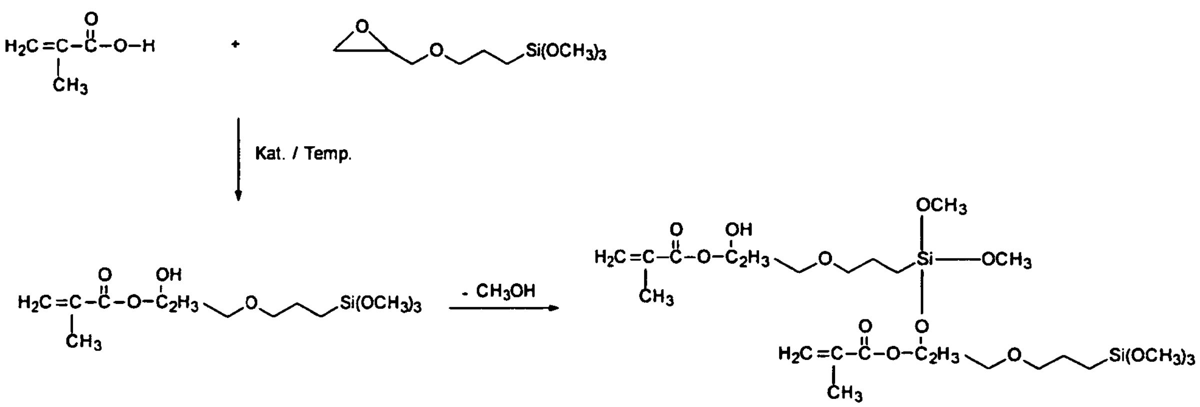


Zur Vorlage von 199,1 g (0,802 mol) 3-Glycidyloxypropyltrimethoxysilan werden unter trockener Atmosphäre (Sauerstoff) Triphenylphosphin als Katalysator, BHT als Stabilisator und anschließend 75,76 g (0,88 mol) Methacrylsäure

zugetropft und bei 85 °C gerührt (ca. 24 h). Die Umsetzung kann über die Abnahme der Carbonsäurekonzentration mittels Säuretitration sowie dem Epoxidumsatz mittels Ramanspektroskopie/Epoxidtitration verfolgt werden. Die für die Epoxidgruppe vom Epoxysilan charakteristische Bande erscheint im Ramanspektrum bei 1256 $cm^{-1}$. Der Epoxid- bzw. Carbonsäureumsatz liegt bei ≥ 99 % bzw. ≥ 89 % (→ da 1 : 1,1 Carbonsäureüberschuss).

2. Stufe: Hydrolyse/Kondensation

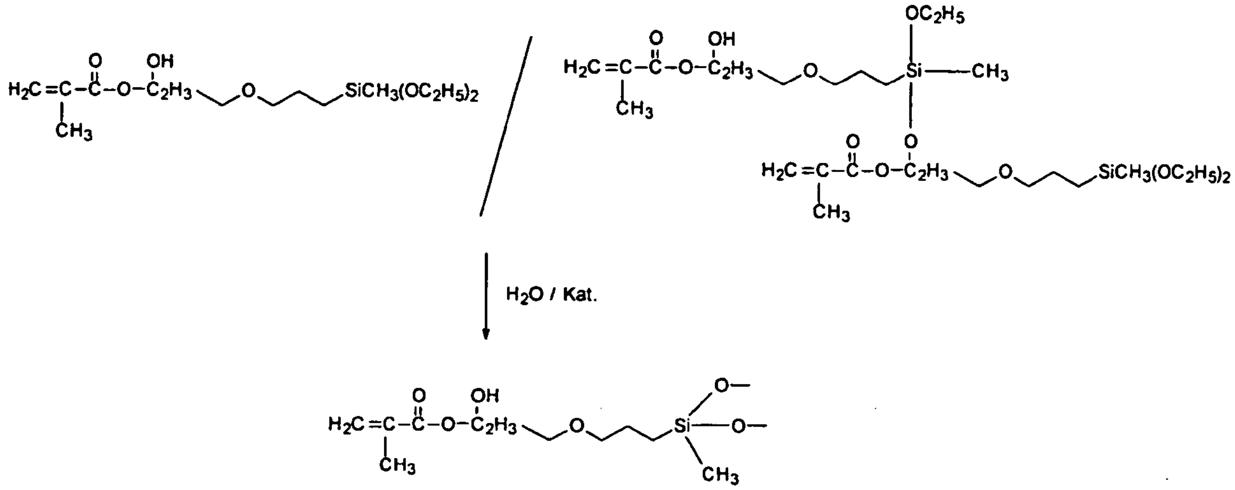


Nach Zugabe von Essigester (1000 ml/mol Silan) und $H_2O$ zur Hydrolyse mit HCl als Kat. wird bei 30 °C gerührt. Der Verlauf der Hydrolyse wird jeweils durch Wassertitration verfolgt. Die Aufarbeitung erfolgt nach ca. mehrtägigem Rühren durch mehrmaliges Ausschütteln mit wässriger NaOH und anschließendem Ausschütteln mit Wasser und Filtration über einen hydrophobierten Filter. Es wird zunächst abrotiert und anschließend mit Ölpumpenvakuum abgezogen, um Alkohol und Wasser zu entfernen. Es resultiert ein Harz, das ohne Einsatz von sogenannten Reaktiwerdünnern (zusätzlichen flüssigen Monomeren) flüssig ist und eine sehr geringe Viskosität (ca. 4-6 Pa·s bei 25 °C (stark abhängig von den genauen Hydrolyse- und Aufarbeitungsbedingungen) besitzt. Der $CO_2H$-Gehalt wurde mit 0,00 mmol/g gemessen, d.h. das Harz enthält keine freien, monomeren Methacrylsäure-Moleküle mehr.

**Beispiel 2**

Dieses Beispiel erläutert die Umsetzung des Harzsystems aus Beispiel 1 mit einem Isocyanat

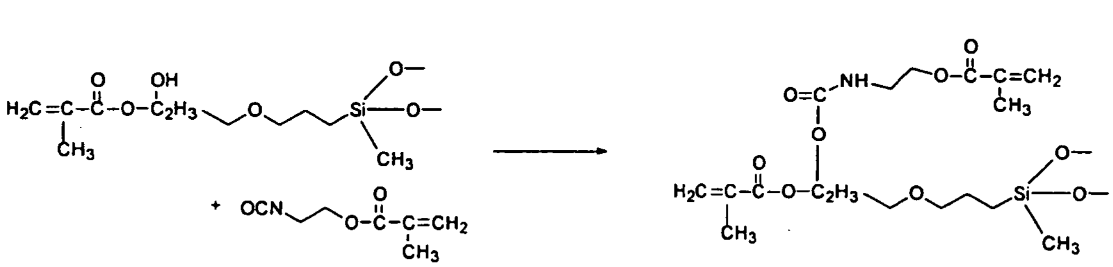

Zur Vorlage von 73,9 g (0,28 mol) obigen Harzes werden unter trockener Atmosphäre (Sauerstoff) bei RT unter Rühren 30,41 g (0,20 mol) Methacrylsäureisocyanatoethylester zugetropft und bei 30 °C gerührt. Die Umsetzung kann über die Abnahme der OCN-Bande mittels IR-Spektrum verfolgt werden. Die für die OCN-Gruppe charakteristische Bande erscheint im IR-Spektrum bei 2272 $cm^{-1}$. Es resultiert ein zähflüssiges Harz mit einer Viskosität von ca. 18 -25

Pa·s bei 25 °C (stark abhängig von den genauen Synthese- und Aufarbeitungsbedingungen insbesondere auch der Vorstufen).
IR-Daten:

$$\nu_{(OH \leftarrow Edukt)} \approx 3500\ cm^{-1}$$

(Rest-OH, da nur mit 0,7 Molanteilen Methacrylsäure-isocyanatöthylester umgesetzt wurde)

$$\nu_{(NH \leftarrow Urethan)} \approx 3373\ cm^{-1}$$

$$\nu_{(C=O \leftarrow Methacrylat/Urethan)} \approx 1721\ cm^{-1}$$

$$\nu_{(C=C \leftarrow Methacrylat)} \approx 1638\ cm^{-1}$$

Nachstehend werden einige Eigenschaften des Matrixsystems gemäß Beispiel 1 (ohne Umsetzung mit einem Isocyanat) mit solchen des Matrixsystems gemäß Beispiel 2 verglichen:

| | Matrixsystem (Beispiel 1) | Matrixsystem (Beispiel 2) |
|---|---|---|
| Bruchfestigkeit [MPa] 1,5 Tage bei 40°C trockengelagert | 70-80 | 103-115 |
| E-Modul [GPa] | 1,7-1,9 | 2,2-2,5 |
| Schrumpfung [Vol-%] (15 min) | 5,4 | 4,2-4,6 |
| Zytotoxizität [% aktive Zellen] nach 24 h | 95-97 | 97-102 |

Der Vergleich zeigt, dass die organische Komponente der erfindungsgemäßen Matrixsysteme verbesserte Eigenschaften im Vergleich zu den im Stand der Technik eingesetzten Harze besitzt: Festigkeit und E-Modul sind deutlich erhöht, und die Schrumpfung ist wesentlich reduziert.

**Beispiel 3 und Vergleichsbeispiele**

**a) Kompositherstellung und Charakterisierung**

Die Einarbeitung der verschiedenen Füllstofftypen in das beschriebene Harzsystem nach Zusatz üblicher Photoinitiatoren sowie weiterer Dentaladditive erfolgt nach üblichen Verfahren durch Einsatz verschiedener Mischertypen bei verschiedenen Temperaturen und ggf. unter Vakuum.

Die resultierenden Komposite werden in eine Stäbchenform (2 x 2 x 25 $mm^3$) gegeben. Die Methacrylatgruppen werden im Rahmen einer photoinduzierten radikalischen Polymerisation umgesetzt, wobei das jeweilige Komposit aushärtet. Mittels 3-Punktbiegeversuch wird nach 1,5 Tagen Lagerung unter Luft bzw. Wasser bei 40 °C das E-Modul sowie die Bruchfestigkeit (bei Raumtemperatur) der resultierenden Stäbchen bestimmt.

Die Schrumpfungswerte der Komposite werden mittels Auftriebsmethode (Komposit 3) bzw. mittels Quecksilberdilatometer (Vergleichskomposite 1 und 2) im Rahmen einer photoinduzierten radikalischen Polymerisation erhalten.

Die in vitro-Zytotoxizitätstestung erfolgt mittels Wachstumsinhibitionstest in der Mikrotiterplatte an 3T3-Maus-Fibroblasten an Kompositeluaten an der MHH in Hannover.

Die Abrasion wurde in einer 3-Medienabrasionsmaschiene nach ACTA-Verfahren bestimmt.

Die Röntgenopazität wurde gemäß EN ISO 4049 mit einem Röntgengerät Typ Polydoros SX80 der Fa. Siemens und Ultraspeed-Filmen der Fa. Kodak gemessen. Die Ermittlung der Schwärzungsgrade erfolgte mittels eines Wellhöfer Dosimeters Lullus 1.21 (Photodensitometer).

Der thermischer Ausdehnungskoeffizient α wurde mit einem Dilatometer der Firma Netzsch 402 E/7 im Temperaturbereich von 5 bis 50 °C gemessen und ausgewertet.

**b) Kompositdaten**

Im folgenden sind die Materialkenndaten von zwei kommerziellen Füllungskompositen (Komposit 1 u. 2) aufgeführt, im Vergleich zu einem Komposit 3 auf Basis des Matrixsystems gemäß Beispiel 2 in Verbindung mit Standard-Füllstoffen und einem erfindungsgemäßen Komposit 4 auf Basis des Matrixsystems gemäß Beispiel 2 mit nanopartikulären Füllstoffen.

| | Komposit 1 | Komposit 2 | Komposit 3 | Komposit 4 |
|---|---|---|---|---|
| Füllstoffgemisch | Ba-Al-Borosilikatglas/ hochdisperse Kieselsäure | Ba-Al-Borosilikatglas/Ba-Al-Fluorosilikatglas/ Ytterbiumtrifluorid/;Hoch disperse Kieselsäure | Ba-Al-Borosilikatglas, hochdisperse Kieselsäure, alle Füllstoffe silanisiert | Ba-Al-Borosilikatglas, $SiO_2$Partikel (60 nm), $SiO_2/SnO_2$ Kern-Schalepartikel (80 nm), alle silanisiert) |
| Partikelgröße [μm] | 0,04 - 2,0 | 0,04 - 3,0 | 0,04 - 3,0 | 0,06 - 3,0 |
| Füllstoffgehalt [Gew.-%] | 75,0 | 80,0 | 77,0 | 82,0 |
| Füllstoffgehalt [Vol.-%] | 56,5 | 60,0 | 60,4 | 67,7 |
| Biegefestigkeit [MPa] (24 h/ 37°C, gemäß ISO 4049) | 118,9 (± 11,2) | 116,0 (± 12,5) | 132,0 (± 9,0) | 145,0 (± 9,0) |
| E-Modul [MPa] (24 h/37°C, gemäß ISO 4049) | 8150 (± 385) | 8750 (± 360) | 11100 (± 300) | 11300 (± 300) |
| Schrumpfung [Vol.-%]; 30 min. Wert | 2,99 | 3,07 | 2,2 | 1,3 |
| Röntgenopazität [% Al][4] | 220 | 380 | ≤ 250 | 311 |
| Abrasion [5] | 58 | 66 | 48 | 30 |
| Monomere | BISGMA<br>UDMA<br>TEGDMA | BISGMA [1]<br>UDMA[2]<br>TEGDMA[3] | Monomerfrei | Monomerfrei |
| Therm. Ausdehnungskoeffizient $\alpha$(5-50°C) [$10^{-6}K^{-1}$] | 49,8 | 47,5 | n.b. | 26,9 |
| Zytotoxizität, 3T3-Zellen [% vitale Zellen nach Inkubationszeit]: 24 h<br>48 h | 88,5 (± 8,6)<br>93,0 (± 4,1) | 96,6 (± 8,3)<br>86,4 (± 2,1) | 99,4<br>98,6 | 93,0<br>99,6 |

[1] 2,2'-Bis[4-(3'-methacryloyl-oxy-2'-hydroxylpropoxy-phenyl]propan
[2] 7,7,9-Trimethyl-4,13-dioxo-3,14-dioxa-5,12-diazahexadecan-1,16-dioxy-dimethacrylat
[3] Triethylenglycoldimethacrylat
[4] Gemäß ISO 4049
[5] nach ACTA-Verfahren [μm] (3-Medienabrasion)

Das dem Komposit 1 zu Grunde liegende Harzsystem besitzt eine Schrumpfung von > 7%, ist diesbezüglich also deutlich ungünstiger als das Harz des Beispiels 2.

Ein Vergleich der Komposite 3 und 4 zeigt die Vorteile der Einarbeitung eines nanopartikulären Füllstoffanteil in den Komposit: Bei gleicher Harzmatrix ist ein deutlich höherer Füllstoffgehalt möglich, die Festigkeit des Komposits ist höher, die Schrumpfung ist drastisch vermindert, die Röntgenopazität ist deutlich erhöht, und die Abrasion ist deutlich geringer.

Bei Ersatz der $SiO_2$-Partikel (60 nm) in Komposit 4 durch $SiO_2/SnO_2$-Partikel (80 nm) lässt sich die Röntgenopazität von 311 weiter auf 361 % Al steigern.

Der nanopartikuläre Füllstoffanteil bewirkt einen deutlich erhöhten Füllstoffgehalt und dabei insbesondere einen sehr hohen Volumenanteil im Komposit, welches sich dennoch gut applizieren lässt, d. h. ein angepasstes Fließverhalten zeigt. Es resultiert mit 1,3 Vol.-% eine überraschend deutlich reduzierte Schrumpfung in Verbindung mit einem drastisch verringerten Abrasionsverhalten, einer hohen Festigkeit, einem dem Dentin (E-Modul ca. 18 GPa bzw. in Flüssigkeit ca. 10 GPa; $\alpha$ ca. $8{*}10^{-6}$ $K^{-1}$) angenähertem E-Modul sowie angenähertem thermischen Ausdehnungskoeffizienten, einer hohen Röntgenabsorption (> 250 % Al gewünscht), einer guten Transluzenz (wichtig für das optische Erscheinungsbild der Füllung) und ausgezeichneter Biokompatibilität (nach Zytotoxizitätstest). Wie Komposit 4 zeigt, ist eine monomerfreie Ausführung möglich, was z. B. aus Sicht einer allergologische Belastung auch schon während der Applikation, d. h. im ungehärteten Zustand, von besonderer Bedeutung für das zahnärztliche Personal ist.

## Patentansprüche

**1.** Dentalkomposit, umfassend

(A) eine Matrix, umfassend ein Harz mit dem nachfolgenden Strukturelement (Ib),

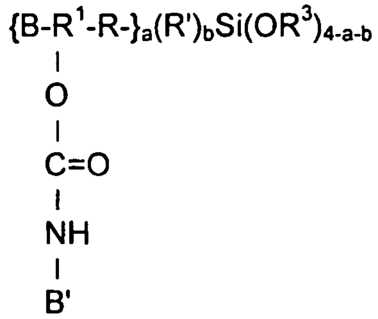

(Ib)

worin die Reste und Indices die folgende Bedeutung haben:

R ist eine offenkettige und/oder cyclische Alkylen-, Arylen- oder Alkylenarylengruppe mit jeweils 1 bis 10 Kohlenstoffatomen, die durch eine oder mehrere Sauerstoff- oder Schwefelatome oder Carboxyl- oder Aminogruppen unterbrochen sein oder solche Atome/Gruppen an ihrem dem Siliciumatom abgewandten Ende tragen kann;
$R^1$ ist eine mit der in der Formel gezeigten Urethangruppe substituierte, offenkettige und/oder cyclische Alkylen-, Arylen- oder Alkylenarylengruppe mit jeweils 1 bis 10 Kohlenstoffatomen, die durch eine oder mehrere Sauerstoff- oder Schwefelatome oder Carboxyl- oder Aminogruppen unterbrochen sein oder solche Atome/Gruppen an einem ihrer Enden tragen kann,
R' ist eine offenkettige und/oder cyclische Alkyl-, Alkenyl-, Aryl-, Alkylaryl- oder Arylalkylgruppe mit vorzugsweise 1 bis 20 Kohlenstoffatomen,
B und B' sind gleich oder verschieden; beide Reste haben die Bedeutung einer geradkettigen oder verzweigten, organisch polymerisierbaren Gruppe mit mindestens einer (Meth-)Acrylatgruppe und damit mindestens 3 Kohlenstoffatomen,
die Reste $R^3$ sind gleich oder verschieden und besitzen zumindest teilweise die Bedeutung einer Bindung zu einem anderen Siliciumatom und stellen im übrigen ein Wasserstoffatom, eine Alkylgruppe mit 1 bis 10 Kohlenstoffatomen oder eine Bindung zu einem anderen Metallatom dar, das sich in Kieselsäureheteropolykondensate einbauen lässt,
a bedeutet 1 oder 2,

und b ist 0 oder 1,

und
(B) einen nanopartikulären Füllstoff.

2. Dentalkomposit nach Anspruch 1, weiterhin umfassend einen Polymerisationsinitiator, vorzugsweise ausgewählt unter Initiatoren für die radikalische Härtung, Initiatoren für die thermische Härtung und Initiatoren für die Strahlungshärtung

3. Dentalkomposit nach Anspruch 1 oder 2, worin B und/oder B' eine oder zwei Methacrylatgruppen enthalten oder ein Methacrylatrest sind.

4. Silan nach einem der voranstehenden Ansprüche, worin die Reste B und optional auch B' Methacrylsäureestergruppen des Trimethylolpropan, der Glycerins, des Pentaerythrits, der $C_2$-$C_4$-Alkandiole, der Polyethylenglycole, der Polypropylenglycole oder des gegebenenfalls substituierten und/oder alkoxylierten Bisphenol A sind oder diese Ester umfassen.

5. Dentalkomposit nach einem der voranstehenden Ansprüche, worin $R^1$ eine urethangruppensubstituierte $C_1$-$C_6$-Alkylengruppe ist.

6. Dentalkomposit nach einem der voranstehenden Ansprüche, worin die Strukturelemente des Harzes zu mindestens 60 Mol-%, vorzugsweise im Wesentlichen und ganz besonders bevorzugt ausschließlich aus solchen der der Formel (Ib) bestehen.

7. Dentalkomposit nach einem der voranstehenden Ansprüche, worin der nanopartikuläre Füllstoff aus annähernd sphärischen Teilchen mit enger Durchmesserverteilung besteht oder solche Teilchen enthält.

8. Dentalkomposit nach Anspruch 7, worin die nanopartikulären Teilchen im Wesentlichen einen Durchmesser zwischen 10 und 200 nm aufweisen.

9. Dentalkomposit nach einem der voranstehenden Ansprüche, worin neben dem nanopartikulären Füllstoff weiterer Füllstoff vorhanden ist.

10. Dentalkomposit nach Anspruch 9, worin der weitere Füllstoff ausgewählt ist unter Makrofüllern aus Glas, Keramik oder Quarz mit Teilchengrößen zwischen 2 bis 50 μm, homogenen Mikrofüllern insbesondere aus pyrogener Kieselsäure, inhomogenen Mikrofüllern, vorzugsweise in Form eines Splitterpolymerisats, insbesondere Mischungen aus pyrogener Kieselsäure und Ba- oder Sr-Glas mit Teilchengrößen im Bereich von etwa 1 bis 5 μm, Dentalgläsern mit Teilchendurchmessern von ca. 1-5 μm, Mischungen dieser Füller, Hybridfüllern oder Feinsthybridfüllern.

11. Dentalkomposit nach einem der voranstehenden Ansprüche, enthaltend zwischen 1 und 50 Gew.-% Gesamt-Füllstoff.

12. Dentalkomposit nach einem der Ansprüche 1 bis 5, enthaltend zwischen 70 und 90 Gew.-% Gesamt-Füllstoff.

13. Dentalkomposit nach einem der voranstehenden Ansprüche, worin zwischen 5 und 60 Gew.-% des Gesamt-Füllstoffs nanopartikulärer Füllstoff sind.

14. Dentalkomposit nach Anspruch 13, worin zwischen 29 und 35 Gew.-% des Gesamt-Füllstoffs nanopartikulärer Füllstoff sind.

15. Dentalmaterial gemäß einem der voranstehenden Ansprüche zur Verwendung als Verbundmaterial, Befestigungsmaterial, Zement, Füllungsmaterial, Adhäsiv, Beschichtungsmaterial, Fissurenversiegler, Zahnhalsbeschichtung, Kronen- oder Brückenmaterial oder Bonding.

## Claims

1. Dental composite comprising

(A) a matrix comprising a resin having the following structural element (Ib)

$$\{B\text{-}R^1\text{-}R\text{-}\}_a(R')_bSi(OR^3)_{4\text{-}a\text{-}b} \qquad (Ib)$$

```
 I
 O
 I
C=O
 I
NH
 I
 B'
```

wherein the radials and indices have the following meaning:

R is an open-chain and/or cyclic alkylene, arylene or alkylene-arylene group having in each case 1 to 10 carbon atoms which may be interrupted by one or more oxygen or sulphur atoms or carboxyl or amino groups or may carry such atoms/groups at its end facing away from the silicon atom;
R1 is an open-chain and/or cyclic alkylene, arylene or alkylene-arylene group substituted by the urethane group shown in the formula and having in each case 1 to 10 carbon atoms, which may be interrupted by one or more oxygen or sulphur atoms or carboxyl or amino groups or may carry such atoms/groups at one of its ends,
R' is an open-chain and/or cyclic alkyl, alkenyl, aryl, alkylaryl or arylalkyl group having preferably 1 to 20 carbon atoms,
B and B' are the same or different; both radicals have the meaning of a straight-chain or branched, organically polymerisable group having at least one (meth)acrylate group and hence at least 3 carbon atoms,
the radicals R3 are the same or different and have at least in some cases the meaning of a bond to a further silicon atom and otherwise represent a hydrogen atom, an alkyl group having 1 to 10 carbon atoms or a bond to a further metal atom, which can be incorporated in silicic acid heteropolycondensates,
a denotes 1 or 2,
and b is 0 or 1,

and
(B) a nanoparticulate filler.

2. Dental composite according to claim 1, further comprising a polymerisation initiator, preferably selected from initiators for free-radical curing, initiators for thermal curing and initiators for radiation curing.

3. Dental composite according to claim 1 or 2, wherein B and/or B' contain one or two methacrylate groups or are a methacrylate radical.

4. Silane according to one of the preceding claims, wherein the radicals B and optionally also B' are methacrylate groups of trimethylol propane, of glycerol, of pentaerythritol, of $C_2$-$C_4$ alkane diols, of polyethylene glycols, of polypropylene glycols or of optionally substituted and/or alkoxylated bisphenol A or comprise these esters.

5. Dental composite according to one of the preceding claims, wherein $R^1$ is a urethane group-substituted $C_1$-$C_6$ alkylene group.

6. Dental composite according to one of the preceding claims, wherein the structural elements of the resin consist to at least 60 mole%, preferably essentially and most particularly preferably exclusively of those of formula (Ib).

7. Dental composite according to one of the preceding claims, wherein the nanoparticulate filler consists of approximately spherical particles with narrow diameter distribution or contains such particles.

8. Dental composite according to claim 7, wherein the nanoparticulate particles have essentially a diameter between 10 and 200 nm.

**9.** Dental composite according to one of the preceding claims, wherein in addition to the nanoparticulate filler, further filler is present.

**10.** Dental composite according to claim 9, wherein the further filler is selected from macro-fillers made from glass, ceramic or quartz having particle sizes between 2 to 50 μm, homogeneous micro-fillers in particular made from pyrogenic silicic acid, inhomogeneous micro-fillers, preferably in the form of a chipped polymer, in particular mixtures of pyrogenic silicic acid and Ba or Sr glass having particle sizes in the range from about 1 to 5 μm, dental glasses having particle diameters of about 1-5 μm, mixtures of these fillers, hybrid fillers or very fine hybrid fillers.

**11.** Dental composite according to one of the preceding claims, containing between 1 and 50 wt.% of total filler.

**12.** Dental composite according to one of claims 1 to 5, containing between 70 and 90 wt.% of total filler.

**13.** Dental composite according to one of the preceding claims, wherein between 5 and 60 wt.% of the total filler is nanoparticulate filler.

**14.** Dental composite according to claim 13, wherein between 29 and 35 wt.% of the total filler is nanoparticulate filler.

**15.** Dental material according to one of the preceding claims for use as composite material, fixing material, cement, filling material, adhesive, coating material, fissure sealant, dental neck coating, crown or bridge material or bonding.

**Revendications**

**1.** Composite dentaire comprenant :

(A) une matrice, contenant une résine ayant l'élément structural (Ib) suivant :

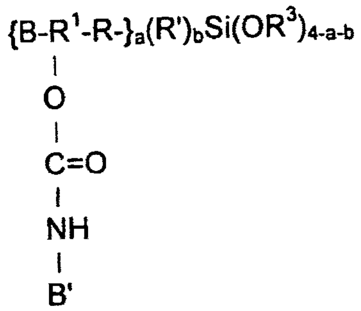

(Ib)

dans lequel les radicaux et les indices ont la signification suivante :

R désigne un groupe alkylène, arylène ou alkylène-arylène à chaîne ouverte et/ou cyclique ayant respectivement 1 à 10 atomes de carbone qui est coupé par un ou plusieurs atomes d'oxygène ou de soufre ou bien par un ou plusieurs groupes carboxyliques ou amino ou encore qui peut porter ces atomes/groupes sur son extrémité éloignée de l'atome de silicium ;
$R^1$ désigne un groupe alkylène, arylène ou alkylène-arylène à chaîne ouverte et/ou cyclique ayant respectivement 1 à 10 atomes de carbone, substitué par un groupe uréthane indiqué dans la formule (Ib), qui est coupé par un ou plusieurs atomes d'oxygène ou de soufre ou bien par des groupes carboxyliques ou amino ou encore qui peut porter ces atomes/groupes sur une de ses extrémités ;
R' est un groupe alkyle, alcényle, aryle, alkylaryle ou arylalkyle à chaîne ouverte et/ou cyclique ayant de préférence 1 à 20 atomes de carbone,
B et B' sont identiques ou différents ; les deux radicaux désignent un groupe polymérisable par voie organique à chaîne droite ou ramifiée ayant au moins un groupe (méth)acrylate et au moins 3 atomes de carbone,
les radicaux $R^3$ sont identiques ou différents et désignent au moins partiellement une liaison à un autre atome de silicium et représentent en outre un atome d'hydrogène, un groupe alkyle ayant 1 à 10 atomes de carbone ou une liaison à un autre atome de métal qui peut être incorporé dans des hétéropolycondensats d'acide silicique,

a désigne 1 ou 2,
et b vaut 0 ou 1,

et
(B) est une charge nanoparticulaire.

**2.** Composite dentaire selon la revendication 1, comprenant en outre un initiateur de polymérisation, choisi de préférence parmi les initiateurs de durcissement par voie radicalaire, les initiateurs de durcissement par voie thermique et les initiateurs de durcissement par rayonnement.

**3.** Composite dentaire selon la revendication 1 ou 2, dans lequel B et/ou B' contiennent un ou deux groupes méthacrylate ou sont un radical méthacrylate.

**4.** Silane selon l'une des revendications précédentes, dans lequel les radicaux B et éventuellement B' comprennent des groupes esters de l'acide méthacrylique du triméthylolpropane, de la glycérine, du pentaérythritol, des alcanediols en $C_2$-$C_4$, des polyéthylène glycols, des polypropylène glycols ou du bisphénol A éventuellement substitué et/ou alcoxylé ou bien comprennent ces esters.

**5.** Composite dentaire selon l'une des revendications précédentes, dans lequel $R^1$ est un groupe alkylène en $C_1$-$C_6$ substitué par un groupe uréthane.

**6.** Composite dentaire selon l'une des revendications précédentes, dans lequel les éléments structuraux de la résine sont constitués d'au moins 60 % molaires, de préférence sensiblement ou de manière particulièrement préférée exclusivement, de ces éléments structuraux de formule (Ib).

**7.** Composite dentaire selon l'une des revendications précédentes, dans lequel la charge nanoparticulaire est constituée de particules à peu près sphériques ayant une distribution de diamètre étroite ou bien contient ce type de particules.

**8.** Composite dentaire selon la revendication 7, dans lequel les particules nanoparticulaires présentent sensiblement un diamètre compris entre 10 et 200 nm.

**9.** Composite dentaire selon l'une des revendications précédentes, dans lequel une autre charge est présente outre la charge nanoparticulaire.

**10.** Composite dentaire selon la revendication 9, dans lequel l'autre charge est choisie parmi les macrocharges de verre, de céramique ou de quartz ayant une granulométrie comprise entre 2 et 50 $\mu$m, les microcharges homogènes, notamment parmi la silice pyrogénée, les microcharges hétérogènes, de préférence sous forme de polymère pulvérulent, notamment des mélanges de silice pyrogénée et de verre Ba ou Sr ayant une granulométrie approximative de 1 à 5 $\mu$m, les verres dentaires ayant des diamètres de particules d'environ 1 à 5 $\mu$m, les mélanges de ces charges, les charges hybrides ou les charges hybrides très fines.

**11.** Composite dentaire selon l'une des revendications précédentes, contenant entre 1 et 50 % en poids de charge totale.

**12.** Composite dentaire selon l'une des revendications 1 à 5, contenant entre 70 et 90 % en poids de charge totale.

**13.** Composite dentaire selon l'une des revendications précédentes, dans lequel la charge nanoparticulaire représente entre 5 et 60 % en poids de la charge totale.

**14.** Composite dentaire selon la revendication 13, dans lequel la charge nanoparticulaire représente entre 29 et 35 % en poids de la charge totale.

**15.** Matériau dentaire selon l'une des revendications précédentes destiné à être utilisé comme composite, fixateur, ciment, charge, adhésif, revêtement, matériau pour scellement des puits et des fissures, revêtement du collet de la dent, matériau pour couronne ou bridge ou collage.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10349766
- DE 4133494
- WO 0108639 A1
- DE 4416857 C1
- DE 4416857 A
- DE 4125201 C1
- DE 19643781
- DE 10041038
- DE 10018405
- DE 19903177 C2
- DE 10348766


### In der Beschreibung aufgeführte Nicht-Patentliteratur


- *Jahrestätigkeitsberichts des Fraunhofer Instituts für Silicatforschung,* 1992, 61-72
- *Polymer + Materials Research Symposium,* 1993, 14-17
- *Journal f. prakt. Chemie,* 1988, vol. 330, 316-318